(19) Europäisches Patentamt
European Patent Office
Office européen des brevets

(11) **EP 4 312 981 B1**

(12) **EUROPEAN PATENT SPECIFICATION**


(45) Date of publication and mention of the grant of the patent:
**05.11.2025 Bulletin 2025/45**

(21) Application number: **22720894.9**

(22) Date of filing: **25.03.2022**

(51) International Patent Classification (IPC):
***A61K 9/00*** *(2006.01)* ***A61K 9/68*** *(2006.01)*
***A61K 47/02*** *(2006.01)* ***A61K 47/10*** *(2017.01)*
***A61K 47/18*** *(2017.01)*

(52) Cooperative Patent Classification (CPC):
**A61K 9/0056; A61K 9/0058; A61K 47/02; A61K 47/10; A61K 47/18**

(86) International application number:
**PCT/US2022/071335**

(87) International publication number:
**WO 2022/204715 (29.09.2022 Gazette 2022/39)**


(54) **EDIBLE ORAL COMPOSITIONS COMPRISING HOPS**

ESSBARE ORALE ZUSAMMENSETZUNGEN MIT HOPFEN

COMPOSITIONS ORALES COMESTIBLES COMPRENANT DU HOUBLON


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2021 US 202163165748 P**

(43) Date of publication of application:
**07.02.2024 Bulletin 2024/06**



(73) Proprietor: **The Procter & Gamble Company**
**Cincinnati, OH 45202 (US)**

(72) Inventor: **ST. JOHN, Samuel, James**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**



(56) References cited:
**WO-A1-2015/171837** **WO-A1-99/09842**
**US-A- 6 165 447**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 4 312 981 B1

## Description

### FIELD OF THE INVENTION

The present invention is directed to edible oral compositions comprising hops.

### BACKGROUND OF THE INVENTION

In many jurisdictions, the only active agent that is approved as an anticavity drug are compounds including fluoride, such as stannous fluoride, sodium fluoride, amine fluoride, and/or sodium monofluorophosphate. Many of these compounds are included in oral compositions. Oral compositions including fluoride can, in many jurisdictions, make anticavity or anticaries drug claims.

Fluoride supplements, according to the United States Food & Drug Administration, are intended to be swallowed because they have a lower concentration of fluoride ions (up to 0.7 ppm). Fluoride supplements include fluoridated water and specialty low concentration rinses intended only for individuals that do not have access to fluoridated water.

Compositions including anticaries drugs, such as fluoride, in therapeutic doses are not intended to be swallowed. As such, edible compositions, such as chews, lollipops, mints, and gummies, are not permitted to provide anticaries benefits.

Patent WO 2015/171837A1 discloses oral care compositions having improved efficacy for preventing and/or reducing caries comprising xylitol and hops.

Thus, there is a need for one or more active ingredients that have been clinically shown to provide subtherapeutic and/or therapeutic anticaries activity, but can be intentionally swallowed.

### SUMMARY OF THE INVENTION

The invention discloses an edible oral composition comprising:

(a) hops, preferably wherein the edible oral composition comprises from about 0.01% to about 10%, by weight of the oral composition, of the hops, wherein the hops comprises hops beta acid and wherein the hops beta acid is free of hydrogentated hops beta acid; and

(b) calcium, wherein the calcium comprises calcium salt, calcium abrasive, or combinations thereof, wherein the calcium abrasive comprises calcium carbonate, calcium pyrophosphate, or combinations thereof, and wherein the calcium salt comprises calcium chloride, calcium citrate, or combinations thereof.

The edible oral composition further comprises tin, and/or sugar alcohol.

### DETAILED DESCRIPTION OF THE INVENTION

The present invention is directed to edible compositions, as disclosed in the appended claims, comprising hops for the treatment of cavities, caries, gingivitis, and/or combinations thereof. The disclosed compositions comprising hops can be used in the treatment, reduction, or prevention of caries.

Dental caries, or tooth decay, is a breakdown of the teeth due to the acids made by bacteria. Cavities are caused by the acid produced by bacteria dissolving the hard tissues of the teeth, such as enamel, dentin, and/or cementum. Acid is produced by the bacteria when the bacteria breaks down food debris or fermentable carbohydrates on the tooth's surface.

Fluoride works by making the tooth's surface less soluble to the acid produced by the bacteria, "plaque acid." Tooth's enamel is made from hydroxyapatite ($Ca_5(PO_4)_3(OH)$). Hydroxyapatite can be dissolved from the enamel at a pH of under 5.5 (demineralization). If hydroxyapatite is demineralized in the presence of fluoride ions, fluorapatite ($Ca_5(PO_4)_3(,F)$) can remineralize on the surface of a tooth's enamel. In sum, this process is a replacement of a hydroxyl (OH) ion with a fluoride (F) ion. Fluorapatite is inherently less soluble than hydroxyapatite, even under acidic conditions. Thus, fluoride works as an anticaries drug to make the tooth's surface more resistant and less soluble to plaque acid.

While not wishing to being bound by theory, it is believed that the disclosed oral compositions have a different mechanism of action than fluoride ion therapy. In contrast to the single symptom treatment of fluoride (i.e. treating the result of plaque acid), the disclosed compositions are believed to have anticavity activity through a combination of effects that collectively lead to an anticavity effect. While not wishing to being bound by theory, it is believed that the disclosed oral compositions have an anticavity effect by providing one or more of the following outcomes: (1) suppressing plaque acid production, and/or (2) helping to increase saliva flow and flush residual food debris from the mouth.

The suppression of plaque acid production can be accomplished by providing one or more antibacterial agents to kill the source of the plaque acid (i.e. the bacteria itself) and/or providing one or more biofilm modifiers to disrupt and embed

antibacterial agents within the biofilm matrix.

Helping to increase the saliva flow and flush residual food debris from the mouth can be achieved by the action of sucking, chewing, and/or masticating the edible composition for a period of time.

While each of these mechanisms might not be enough, on its own, to provide an anticavity benefit equivalent to a therapeutic dose of fluoride, in combination these mechanisms can provide an anticavity benefit. As such, the present invention is directed to compositions and methods of use of the compositions that can be fluoride-free, yet still provide an anticavity and/or anticaries benefit. An additional advantage of the disclosed compositions is that the disclosed compositions can be routinely consumed, unlike therapeutic doses of fluoride. Oral care compositions including fluoride include instructions that direct users to not swallow the oral care compositions including fluoride. The disclosed compositions can be freely swallowed if desired, yet still provide anticavity, anticaries, and/or antigingivitis benefits.

Definitions

To define more clearly the terms used herein, the following definitions are provided. Unless otherwise indicated, the following definitions are applicable to this disclosure. If a term is used in this disclosure but is not specifically defined herein, the definition from the IUPAC Compendium of Chemical Terminology, 2nd Ed (1997), can be applied, as long as that definition does not conflict with any other disclosure or definition applied herein, or render indefinite or non-enabled any claim to which that definition is applied.

The term "oral care composition", as used herein, includes a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, toothpaste, tooth gel, subgingival gel, mouth rinse, mousse, foam, mouth spray, lozenge, chewable tablet, chewing gum, tooth whitening strips, floss and floss coatings, breath freshening dissolvable strips, or denture care or adhesive product. The oral care composition may also be incorporated onto strips or films for direct application or attachment to oral surfaces.

The term "edible oral composition, as used herein, includes a product, which in the ordinary course of usage, can be intentionally swallowed for purposes of systemic administration of particular therapeutic agents, and retained in the oral cavity for a time sufficient to contact dental surfaces or oral tissues. Examples of edible oral compositions include dentifrice, toothpaste, tooth gel, subgingival gel, mouth rinse, mousse, foam, mouth spray, lozenge, chewable tablet, chewing gum, tooth whitening strips, floss and floss coatings, breath freshening dissolvable strips, denture care or adhesive product, gummy compositions, chewable compositions, pan-chew composition, tablet compositions, dissolving tablet compositions, dissolving films, leave-on compositions, lollipop compositions, lozenge compositions, nonwoven fiber compositions, soluble foam compositions, liquid compositions, paste compositions, chewing gum composition, or combinations thereof.

"Active and other ingredients" useful herein may be categorized or described herein by their cosmetic and/or therapeutic benefit or their postulated mode of action or function. However, it is to be understood that the active and other ingredients useful herein can, in some instances, provide more than one cosmetic and/or therapeutic benefit or function or operate *via* more than one mode of action. Therefore, classifications herein are made for the sake of convenience and are not intended to limit an ingredient to the particularly stated function(s) or activities listed.

The term "orally acceptable carrier" comprises one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy.

The term "substantially free" as used herein refers to the presence of no more than 0.05%, preferably no more than 0.01%, and more preferably no more than 0.001%, of an indicated material in a composition, by total weight of such composition.

The term "essentially free" as used herein means that the indicated material is not deliberately added to the composition, or preferably not present at analytically detectable levels. It is meant to include compositions whereby the indicated material is present only as an impurity of one of the other materials deliberately added.

As used herein, the articles "a" and "an" are understood to mean one or more of the material that is claimed or described, for example, "an edible oral composition" or "a bleaching agent."

All measurements referred to herein are made at about 23 °C (i.e. room temperature) unless otherwise specified.

Generally, groups of elements are indicated using the numbering scheme indicated in the version of the periodic table of elements published in Chemical and Engineering News, 63(5), 27, 1985. In some instances, a group of elements can be indicated using a common name assigned to the group; for example, alkali metals for Group 1 elements, alkaline earth metals for Group 2 elements, and so forth.

Several types of ranges are disclosed in the present invention. When a range of any type is disclosed or claimed, the intent is to disclose or claim individually each possible number that such a range could reasonably encompass,

including end points of the range as well as any sub-ranges and combinations of sub-ranges encompassed therein.

The term "about" means that amounts, sizes, formulations, parameters, and other quantities and characteristics are not and need not be exact, but can be approximate and/or larger or smaller, as desired, reflecting tolerances, conversion factors, rounding off, measurement errors, and the like, and other factors known to those of skill in the art. In general, an amount, size, formulation, parameter or other quantity or characteristic is "about" or "approximate" whether or not expressly stated to be such. The term "about" also encompasses amounts that differ due to different equilibrium conditions for a composition resulting from a particular initial mixture. Whether or not modified by the term "about," the claims include equivalents to the quantities. The term "about" can mean within 10% of the reported numerical value, preferably within 5% of the reported numerical value.

The edible oral composition can be in any suitable form, such as a solid, liquid, powder, paste, or combinations thereof. The edible oral composition can be dentifrice, tooth gel, subgingival gel, mouth rinse, mousse, foam, mouth spray, lozenge, chewable tablet, chewing gum, tooth whitening strips, floss and floss coatings, breath freshening dissolvable strips, denture care or adhesive product, gummy compositions, chewable compositions, pan-chew composition, dissolving tablet compositions, dissolving films, leave-on compositions, lollipop compositions, lozenge compositions, nonwoven fiber compositions, soluble foam compositions, liquid compositions, paste compositions, chewing gum composition, or combinations thereof.

The components of the dentifrice composition can be incorporated into a film, a strip, a foam, or a fiber-based dentifrice composition. The edible oral composition can include a variety of active and inactive ingredients, such as, for example, but not limited to hops, a tin ion source, a calcium ion source, water, a fluoride ion source, zinc ion source, one or more polyphosphates, humectants, surfactants, other ingredients, and the like, as well as any combination thereof, as described below.

Section headers are provided below for organization and convenience only. The section headers do not suggest that a compound cannot be within more than one section. In fact, compounds can fall within more than one section. For example, stannous chloride can be both a tin ion source and a biofilm modifier, stannous fluoride can be both a tin ion source and a fluoride ion source, glycine can be an amino acid, a buffering agent, and/or a biofilm modifier, among numerous other compounds that can fit amongst several categories and/or sections.

*Humulus lupulus*

The edible oral compositions of the present invention comprise hops. The edible oral compositions can comprise at least one hops compound from Formula I and/or Formula IV. The compound from Formula I and/or Formula IV can be provided by any suitable source, such as an extract from *Humulus lupulus* or Hops, *Humulus lupulus* itself, a synthetically derived compound, and/or salts, prodrugs, or other analogs thereof. The hops extract can comprise one or more hops alpha acids, one or more hops iso-alpha acids, one or more hops beta acids, one or more hops oils, one or more flavonoids, one or more solvents, and/or water. Suitable hops alpha acids (generically shown in Formula I) can include humulone (Formula II), adhumulone, cohumulone, posthumulone, prehumulone, and/or mixtures thereof. Suitable hops iso-alpha acids can include cis-isohumulone and/or trans-isohumulone. The isomerization of humulone into cis-isohumulone and trans-isohumulone can be represented by Formula III.

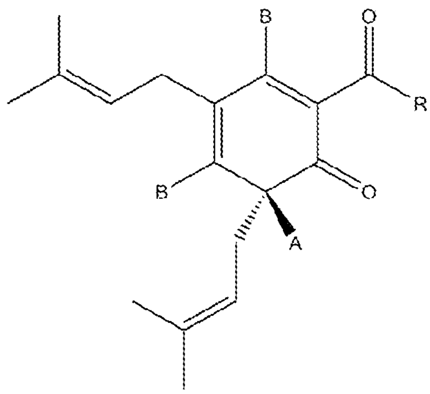

Formula I. Hops Alpha Acids. A is the acidic hydroxyl functional group in the alpha position, B are the acidic hydroxyl functional groups in the beta position, and R is an alkyl functional group.

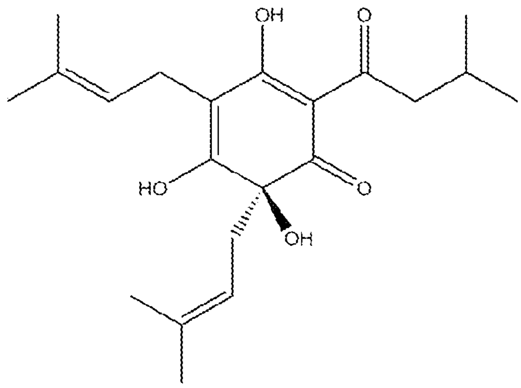

Formula II. Humulone

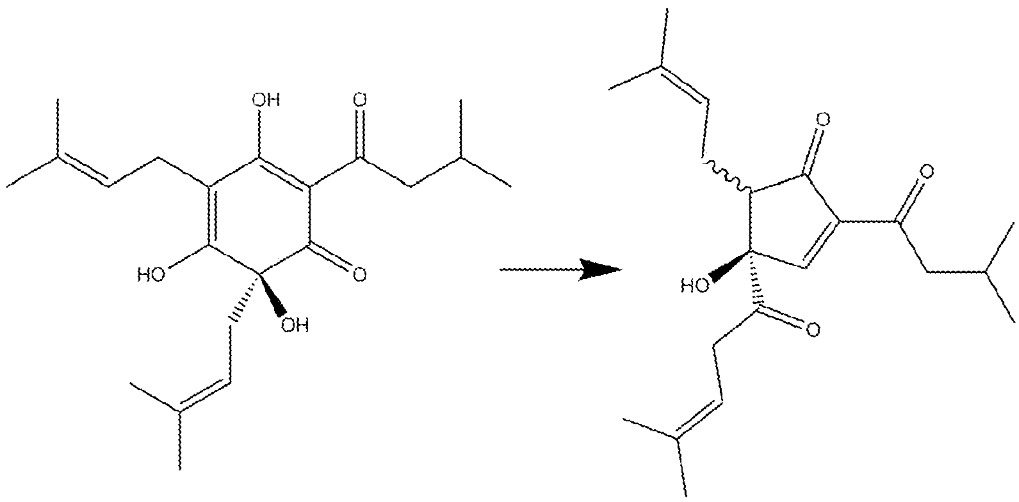

Formula III. Isomerization of Humulone to isohumulone.

Suitable hops beta acids can include lupulone, adlupulone, colupulone, and/or mixtures thereof. A suitable hops beta acid can include a compound a described in Formula IV, V, VI, and/or VII.

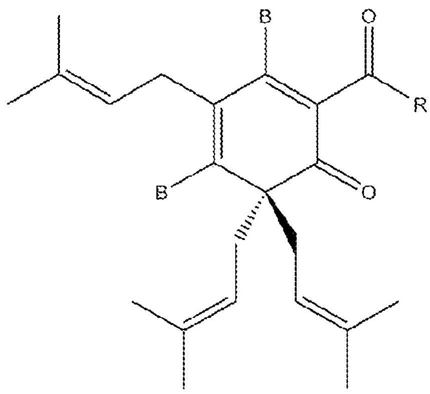

Formula IV. Hops Beta Acids. B are the acidic hydroxyl functional groups in the beta position and R is an alkyl functional group.

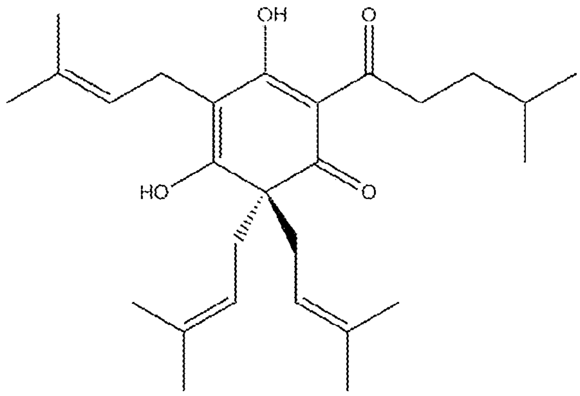

Formula V. Lupulone

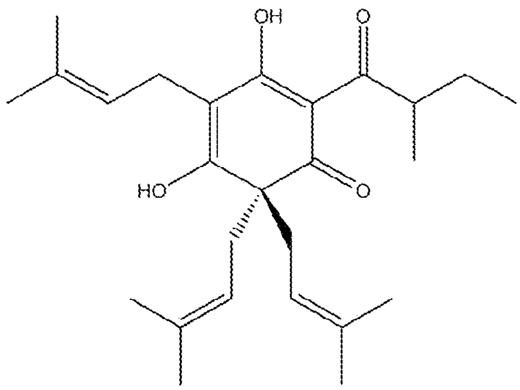

Formula VI. Adlupulone

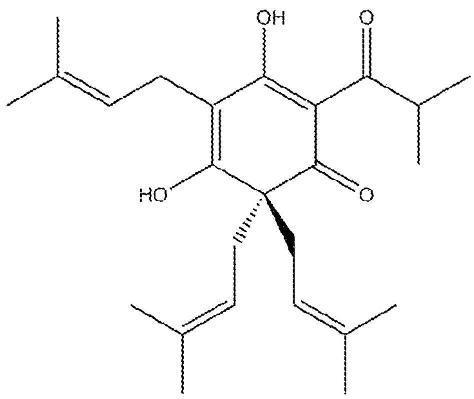

Formula VII. Colupulone

While hops alpha acids can demonstrate some antibacterial activity, hops alpha acids also have a bitter taste. The bitterness provided by hops alpha acids can be suitable for beer, but are not suitable for use in edible oral compositions. In contrast, hops beta acids can be associated with a higher antibacterial and/or anticaries activity, but not as bitter a taste. Thus, a hops extract with a higher proportion of beta acids to alpha acids than normally found in nature, can be suitable for use in edible oral compositions for use as an antibacterial and/or anticaries agent.

A natural hops source can comprise from about 2% to about 12%, by weight of the hops source, of hops beta acids depending on the variety of hops. Hops extracts used in other contexts, such as in the brewing of beer, can comprise from about 15% to about 35%, by weight of the extract, of hops beta acids. The hops extract desired herein can comprise at least about 35%, at least about 40%, at least about 45%, from about 35% to about 95%, from about 40% to about 90%, or from about 45% to about 99%, of hops beta acids. The hops beta acids can be in an acidic form (i.e. with attached hydrogen atom(s) to the hydroxl functional group(s)) or as a salt form.

A suitable hops extract is described in detail in U.S. Patent No. 7,910 140. The hops beta acids desired can be non-hydrogenated, partially hydrogenated by a non-naturally occurring chemical reaction, or hydrogenated by a non-naturally occurring chemical reaction. The hops beta acid can be essentially free of or substantially free of hydrogenated hops beta acid and/or hops acid. A non-naturally occurring chemical reaction is a chemical reaction that was conducted with the aid of chemical compound not found within *Humulus lupulus,* such as a chemical hydrogenation reaction conducted with high heat not normally experienced by *Humulus lupulus* in the wild and/or a metal catalyst.

A natural hops source can comprise from about 2% to about 12%, by weight of the hops source, of hops alpha acids. Hops extracts used in other contexts, such as in the brewing of beer, can comprise from about 15% to about 35%, by weight of the extract, of hops alpha acids. The hops extract desired herein can comprise less than about 10%, less than about 5%, less than about 1%, or less than about 0.5%, by weight of the extract, of hops alpha acids.

Hops oils can include terpene hydrocarbons, such as myrcene, humulene, caryophyllene, and/or mixtures thereof. The hops extract desired herein can comprise less than 5%, less than 2.5%, or less than 2%, by weight of the extract, of one or more hops oils.

Flavonoids present in the hops extract can include xanthohumol, 8-prenylnaringenin, isoxanthohumol, and/or mixtures thereof. The hops extract can be substantially free of, essentially free of, free of, or have less than 250 ppm, less than 150 ppm, and/or less than 100 ppm of one or more flavonoids.

As described in U.S. Patent No. 5,370,863, hops acids have been previously added to edible oral compositions. However, the edible oral compositions taught by U.S. Patent No. 5,370,863 only included up to 0.01%, by weight of the edible oral composition. While not wishing to be bound by theory, it is believed that U.S. Patent No. 5,370,863 could only incorporate a low amount of hops acids because of the bitterness of hops alpha acids. A hops extract with a low level of hops alpha acids would not have this concern.

The hops compound can be combined with or free from an extract from another plant, such as a species from genus *Magnolia.* The hops compounds can be combined with or free from triclosan.

The edible oral composition can comprise from about 0.01% to about 10%, greater than 0.01% to about 10%, from about 0.05%, to about 10%, from about 0.1% to about 10%, from about 0.2% to about 10%, from about 0.2% to about 10%, from about 0.2% to about 5%, from about 0.25% to about 2%, from about 0.05% to about 2%, or from greater than 0.25% to about 2%, of hops beta acid, as described herein. The hops beta acids can be provided by a suitable hops extract, the hops plant itself, or a synthetically derived compound. The hops beta acid can be provided as neutral, acidic compounds, and/or as salts with a suitable counter ion, such as sodium, potassium, ammonia, or any other suitable counter ion.

The hops beta acid can be provided by a hops extract, such as an extract from *Humulus lupulus* with at least 35%, by weight of the extract, of hops beta acid and less than 1%, by weight of the hops extract, of hops alpha acid. The edible oral composition can comprise 0.01% to about 10%, greater than 0.01% to about 10%, from about 0.05%, to about 10%, from about 0.1% to about 10%, from about 0.2% to about 10%, from about 0.2% to about 10%, from about 0.2% to about 5%, from about 0.25% to about 2%, from about 0.05% to about 2%, or from greater than 0.25% to about 2%, of hops extract, as described herein.

Fluoride Ion Source

The edible oral composition can comprise fluoride, such as from a fluoride ion source. The fluoride ion source can comprise one or more fluoride containing compounds, such as stannous fluoride, sodium fluoride, titanium fluoride, calcium fluoride, calcium phosphate silicate fluoride, potassium fluoride, amine fluoride, sodium monofluorophosphate, zinc fluoride, and/or mixtures thereof.

The fluoride ion source and the tin ion source can be the same compound, such as for example, stannous fluoride, which can generate tin ions and fluoride ions. Additionally, the fluoride ion source and the tin ion source can be separate compounds, such as when the tin ion source is stannous chloride and the fluoride ion source is sodium monofluorophosphate or sodium fluoride.

The fluoride ion source and the zinc ion source can be the same compound, such as for example, zinc fluoride, which can generate zinc ions and fluoride ions. Additionally, the fluoride ion source and the zinc ion source can be separate compounds, such as when the zinc ion source is zinc phosphate and the fluoride ion source is stannous fluoride.

The fluoride ion source can be essentially free of or free of stannous fluoride. Thus, the edible oral composition can comprise sodium fluoride, potassium fluoride, amine fluoride, sodium monofluorophosphate, zinc fluoride, and/or mixtures thereof.

The edible oral composition can comprise a fluoride ion source capable of providing from about 50 ppm to about 5000 ppm, and preferably from about 500 ppm to about 3000 ppm of free fluoride ions. To deliver the desired amount of fluoride ions, the fluoride ion source may be present in the edible oral composition at an amount of from about 0.0025% to about 5%, from about 0.01% to about 10%, from about 0.2% to about 1%, from about 0.5% to about 1.5%, or from about 0.3% to about 0.6%, by weight of the edible oral composition. Alternatively, the edible oral composition can comprise less

than 0.1%, less than 0.01%, be essentially free of, substantially free of, or free of a fluoride ion source.

Polyol

The edible oral composition can comprise polyol. A polyol is an organic compound with more than one hydroxyl functional group. The polyol can be a sugar alcohol, a monosaccharide, a disaccharide, a polysaccharide, or a non-reducing sugar. The polyol can be free of, essentially free of, or substantially free of cariogenic sugar molecules.

The polyol can comprise non-reducing sugar. Non-reducing sugars are a class of saccharides that do not generate any compounds containing an aldehyde functional group. Non-reducing sugars are stable in water and do not react with weak oxidizing agents to produce sugar alcohols. Examples of suitable non-reducing sugars include, but are not limited to, sucrose, trehalose, raffinose, stachyose, verbascose, or combinations thereof.

The polyol can comprise sugar alcohol. Sugar alcohols are a class of polyols that can be obtained through the hydrogenation of sugar compounds with the formula $(CHOH)_nH_2$, preferably where n=3-15.

The sugar alcohol can be glycerin, erythritol, xylitol, sorbitol, mannitol, lactitol, galactitol, isomalt, maltitol, maltotriitol, maltotetraitol, polyglycitol, or combinations thereof. Preferably, the stabilizing polyol can be xylitol, sorbitol, galactitol, isomalt, and/or mixtures thereof.

The edible oral composition can comprise 0.01% to about 70%, from about 5% to about 70%, from about 5% to about 50%, from about 10% to about 60%, or from about 20% to about 80%, by weight of the oral care composition, of the polyol.

The sugar alcohol can contribute sweetness, but can be non-cariogenic. Additionally, some sugar alcohols, such as xylitol, have been shown to provide some caries benefits.

Tin Ion Source

The edible oral composition of the present invention can comprise tin, such as from a tin ion source. The tin ion source can be any suitable compound that can provide tin ions in an edible oral composition and/or deliver tin ions to the oral cavity when the dentifrice composition is applied to the oral cavity. The tin ion source can comprise one or more tin containing compounds, such as stannous fluoride, stannous chloride, stannous bromide, stannous iodide, stannous oxide, stannous oxalate, stannous sulfate, stannous sulfide, stannic fluoride, stannic chloride, stannic bromide, stannic iodide, stannic sulfide, and/or mixtures thereof. Tin ion source can comprise stannous fluoride, stannous chloride, and/or mixture thereof. The tin ion source can also be a fluoride-free tin ion source, such as stannous chloride.

The edible oral composition can comprise from about 0.0025% to about 5%, from about 0.01% to about 10%, from about 0.2% to about 1%, from about 0.5% to about 1.5%, or from about 0.3% to about 0.6%, by weight of the edible oral composition, of a tin ion source.

Ca Ion Source

The edible oral composition of the present invention can comprise calcium, such as from a calcium ion source. The calcium ion source can be any suitable compound or molecule that can provide calcium ions in an edible oral composition and/or deliver calcium ions to the oral cavity when the edible oral composition is applied to the oral cavity. The calcium ion source comprises a calcium salt, a calcium abrasive, and/or combinations thereof. In some cases, a calcium salt may also be considered a calcium abrasive or a calcium abrasive may also be considered a calcium salt.

The calcium ion source can comprise a calcium abrasive. The calcium abrasive can be any suitable abrasive compound that can provide calcium ions in an edible oral composition and/or deliver calcium ions to the oral cavity when the edible oral composition is applied to the oral cavity. The calcium abrasive can comprise one or more calcium abrasive compounds, such as calcium carbonate, calcium pyrophosphate, and/or mixtures thereof.

The calcium ion source can comprise a calcium salt, or a compound that can provide calcium ions in an edible oral composition and/or deliver calcium ions to the oral cavity when the edible oral composition is applied to the oral cavity that can not act as an abrasive. The calcium salt can comprise one or more calcium compounds, such as calcium chloride, calcium citrate, and/or combinations thereof.

The edible oral composition can comprise from about 5% to about 70%, from about 10% to about 50%, from about 10% to about 60%, from about 20% to about 50%, from about 25% to about 40%, or from about 1% to about 50% of a calcium ion source.

Buffering Agent

The edible oral composition can comprise a buffering agent. The buffering agent can be a weak acid or base that can maintain a particular pH at a selected site in the oral cavity. For example, the buffering agent can maintain a pH at a

tooth's surface to mitigate the impact of plaque acids produced by bacteria. The buffering agent can comprise a conjugate acid of an ion also present in the edible oral composition. For example, if the calcium ion source comprises calcium carbonate, the buffering agent can comprise a bicarbonate anion ($-HCO_3^-$). The buffering agent can comprise a conjugate acid/base pair, such as citric acid and sodium citrate.

Suitable buffering systems can include phosphate, citrate salts, carbonate/bicarbonate salts, a tris buffer, imidazole, urea, borate, and/or combinations thereof. Suitable buffering agents include bicarbonate salts, such as sodium bicarbonate, glycine, orthophosphate, arginine, urea, and or/combinations thereof.

The edible oral composition can comprise from about 1% to about 30%, from about 5% to about 25% or from about 10% to about 20%, of one or more buffering agents.

Biofilm Modifier

The edible oral composition can comprise one or more biofilm modifiers. A biofilm modifier can comprise a polyol, an ammonia generating compound, and/or a glucosyltransferase inhibitor.

A polyol is an organic compound with more than one hydroxyl functional groups. The polyol can be a sugar alcohol, which area class of polyols that can be obtained through the hydrogenation of sugar compounds with the formula $(CHOH)_nH_2$. The polyol can be glycerin, erythritol, xylitol, sorbitol, mannitol, butylene glycol, lactitol, and/or combinations thereof. The edible oral composition can comprise 0.01% to about 70%, from about 5% to about 70%, from about 5% to about 50%, from about 10% to about 60%, from about 10% to about 25%, or from about 20% to about 80%, by weight of the edible oral composition, of a polyol.

The ammonia generating compound can be any suitable compound that can generate ammonia upon delivery to the oral cavity. Suitable ammonia generating compounds include arginine, urea, and/or combinations thereof. The edible oral composition can comprise from about 0.01% to about 10%, from about 1% to about 5%, or from about 1% to about 25% of one or more ammonia generating compounds.

The glucosyltransferase inhibitor can be any suitable compound that can inhibit a glucosyltransferase. Glucosyltransferases are enzymes that can establish natural glycosidic linkages. In particular, these enzymes break down poly- or oligosaccharide moieties into simple sugars for bacteria associated with dental caries. As such, any compound that can inhibit this process can help prevent dental caries. Suitable glucosyltransferase inhibitors include oleic acid, epicatechin, tannins, tannic acid, moenomycin, caspofungin, ethambutol, lufenuron, and/or combinations thereof. The edible oral composition can comprise from about 0.001% to about 5%, from about 0.01% to about 2%, or about 1% of one or more glucosyltransferase inhibitors.

Metal Ion Source

The edible oral composition can comprise metal, such as from a metal ion source comprising one or more metal ions. The metal ion source can comprise or be in addition to the tin ion source and/or the zinc ion source, as described herein. Suitable metal ion sources include compounds with metal ions, such as, but not limited to Sn, Zn, Cu, Mn, Mg, Sr, Ti, Fe, Mo, B, Ba, Ce, Al, In and/or mixtures thereof. The trace metal source can be any compound with a suitable metal and any accompanying ligands and/or anions.

Suitable ligands and/or anions that can be paired with metal ion sources include, but are not limited to acetate, ammonium sulfate, benzoate, bromide, borate, carbonate, chloride, citrate, gluconate, glycerophosphate, hydroxide, iodide, oxide, propionate, D-lactate, DL-lactate, orthophosphate, pyrophosphate, sulfate, nitrate, tartrate, and/or mixtures thereof.

The edible oral composition can comprise from about 0.01% to about 10%, from about 1% to about 5%, or from about 0.5% to about 15% of a metal ion source.

Antibacterial Agents

The edible oral composition can comprise one or more antibacterial agents. Suitable antibacterial agents include any molecule that provides antibacterial activity in the oral cavity. Suitable antibacterial agents include hops acids, tin ion sources, benzyl alcohol, sodium benzoate, menthylglycyl acetate, menthyl lactate, L-menthol, o-neomenthol, chlorophyllin copper complex, phenol, oxyquinoline, and/or combinations thereof.

The edible oral composition can comprise from about 0.01% to about 10%, from about 1% to about 5%, or from about 0.5% to about 15% of an antibacterial agent.

Bioactive Materials

The edible oral composition can also include bioactive materials suitable for the remineralization of a tooth.

Suitable bioactive materials include bioactive glasses, Novamin™, Recaldent™, hydroxyapatite, one or more amino acids, such as, for example, arginine, citrulline, glycine, lysine, or histidine, or combinations thereof. Suitable examples of compositions comprising arginine are found in U.S. Patent No. 4,154,813 and 5,762,911. Other suitable bioactive materials include any calcium phosphate compound. Other suitable bioactive materials include compounds comprising a calcium source and a phosphate source.

Amino acids are organic compounds that contain an amine functional group, a carboxyl functional group, and a side chain specific to each amino acid. Suitable amino acids include, for example, amino acids with a positive or negative side chain, amino acids with an acidic or basic side chain, amino acids with polar uncharged side chains, amino acids with hydrophobic side chains, and/or combinations thereof. Suitable amino acids also include, for example, arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, omithine, creatine, diaminobutonic acid, diaminoproprionic acid, salts thereof, and/or combinations thereof.

Bioactive glasses are comprising calcium and/or phosphate which can be present in a proportion that is similar to hydroxyapatite. These glasses can bond to the tissue and are biocompatible. Bioactive glasses can include a phosphopeptide, a calcium source, phosphate source, a silica source, a sodium source, and/or combinations thereof.

The edible oral composition can comprise from about 0.01% to about 20%, from about 0.1% to about 10%, or from about 1% to about 10 % of a bioactive material by weight of the edible oral composition.

Abrasive

The edible oral composition can comprise a calcium abrasive, as described herein, and/or a non-calcium abrasive, such as bentonite, silica gel (by itself, and of any structure), precipitated silica, amorphous precipitated silica (by itself, and of any structure as well), hydrated silica, perlite, titanium dioxide, calcium pyrophosphate, dicalcium phosphate dihydrate, alumina, hydrated alumina, calcined alumina, aluminum silicate, insoluble sodium metaphosphate, insoluble potassium metaphosphate, insoluble magnesium carbonate, zirconium silicate, particulate thermosetting resins and other suitable abrasive materials. Such materials can be introduced into the edible oral compositions to tailor the polishing characteristics of the target dentifrice formulation. The edible oral composition can comprise from about 5% to about 70%, from about 10% to about 50%, from about 10% to about 60%, from about 20% to about 50%, from about 25% to about 40%, or from about 1% to about 50%, by weight of the edible oral composition, of the non-calcium abrasive.

Alternatively, the edible oral composition can be substantially free of, essentially free of, or free of silica, alumina, or any other non-calcium abrasive. The edible oral composition can comprise less than about 5%, less than about 1%, less than about 0.5%, less than about 0.1%, or 0% of a non-calcium abrasive, such as silica and/or alumina.

Water

The edible oral composition of the present invention can be anhydrous, a low water formulation, or a high water formulation. In total, the edible oral composition can comprise from 0% to about 99%, from about 5% to about 75%, about 20% or greater, about 30% or greater, or about 50% or greater by weight of the composition, of water. Preferably, the water is USP water.

In a high water edible oral composition and/or toothpaste formulation, the edible oral composition comprises from about 45% to about 75%, by weight of the composition, of water. The high water edible oral composition and/or toothpaste formulation can comprise from about 45% to about 65%, from about 45% to about 55%, or from about 46% to about 54%, by weight of the composition, of water. The water may be added to the high water formulation and/or may come into the composition from the inclusion of other ingredients.

In a low water edible oral composition and/or toothpaste formulation, the edible oral composition comprises from about 5% to about 45%, by weight of the composition, of water. The low water edible oral composition can comprise from about 5% to about 35%, from about 10% to about 25%, or from about 20% to about 25%, by weight of the composition, of water. The water may be added to the low water formulation and/or may come into the composition from the inclusion of other ingredients.

In an anhydrous edible oral composition and/or toothpaste formulation, the edible oral composition comprises less than about 10%, by weight of the composition, of water. The anhydrous composition comprises less than about 5%, less than about 1%, or 0%, by weight of the composition, of water. The water may be added to the anhydrous formulation and/or may come into the composition from the inclusion of other ingredients.

A mouth rinse formulation comprises from about 75% to about 99%, from about 75% to about 95%, or from about 80% to about 95% of water.

The composition can also comprise other orally acceptable carrier materials, such as alcohol, humectants, polymers, surfactants, and acceptance improving agents, such as flavoring, sweetening, coloring and/or cooling agents.

pH

The pH of the disclosed composition can be from about 4 to about 10, from about 7 to about 10, greater than 7 to about 10, greater than 8 to about 10, greater than 7, greater than 7.5, greater than 8, greater than 9, or from about 8.5 to about 10.

Zinc Ion Source

The edible oral composition can comprise zinc, such as from a zinc ion source. The zinc ion source can comprise one or more zinc containing compounds, such as zinc fluoride, zinc lactate, zinc oxide, zinc phosphate, zinc chloride, zinc acetate, zinc hexafluorozirconate, zinc sulfate, zinc tartrate, zinc gluconate, zinc citrate, zinc malate, zinc glycinate, zinc pyrophosphate, zinc metaphosphate, zinc oxalate, and/or zinc carbonate. The zinc ion source can be a fluoride-free zinc ion source, such as zinc phosphate, zinc oxide, and/or zinc citrate.

The zinc ion source may be present in the total edible oral composition at an amount of from about 0.01% to about 10%, from about 0.2% to about 1%, from about 0.5% to about 1.5%, or from about 0.3% to about 0.6%, by weight of the dentifrice composition.

Polyphosphates

The edible oral composition can comprise polyphosphate, such as from a polyphosphate source. A polyphosphate source can comprise one or more polyphosphate molecules. Polyphosphates are a class of materials obtained by the dehydration and condensation of orthophosphate to yield linear and cyclic polyphosphates of varying chain lengths. Thus, polyphosphate molecules are generally identified with an average number (n) of polyphosphate molecules, as described below. A polyphosphate is generally understood to consist of two or more phosphate molecules arranged primarily in a linear configuration, although some cyclic derivatives may be present.

Preferred polyphosphates are those having an average of two or more phosphate groups so that surface adsorption at effective concentrations produces sufficient non-bound phosphate functions, which enhance the anionic surface charge as well as hydrophilic character of the surfaces. Preferred in this invention are the linear polyphosphates having the formula: $XO(XPO_3)_nX$, wherein X is sodium, potassium, ammonium, or any other alkali metal cations and n averages from about 2 to about 21. Alkali earth metal cations, such as calcium, are not preferred because they tend to form insoluble fluoride salts from aqueous solutions comprising a fluoride ions and alkali earth metal cations. Thus, the edible oral compositions disclosed herein can be free of, essentially free of, or substantially free of calcium pyrophosphate.

Some examples of suitable polyphosphate molecules include, for example, pyrophosphate (n=2), tripolyphosphate (n=3), tetrapolyphosphate (n=4), sodaphos polyphosphate (n=6), hexaphos polyphosphate (n=13), benephos polyphosphate (n=14), hexametaphosphate (n=21), which is also known as Glass H. Polyphosphates can include those polyphosphate compounds manufactured by FMC Corporation, ICL Performance Products, and/or Astaris.

The edible oral composition can comprise from about 0.01% to about 15%, from about 0.1% to about 10%, from about 0.5% to about 5%, from about 1 to about 20%, or about 10% or less, by weight of the edible oral composition, of the polyphosphate source.

Humectants

The edible oral composition can comprise one or more humectants, have low levels of a humectant, be essentially free of, be substantially free of, or be free of a humectant. Humectants serve to add body or "mouth texture" to an edible oral composition or dentifrice as well as preventing the dentifrice from drying out. Suitable humectants include polyethylene glycol (at a variety of different molecular weights), propylene glycol, glycerin (glycerol), erythritol, xylitol, sorbitol, mannitol, butylene glycol, lactitol, hydrogenated starch hydrolysates, and/or mixtures thereof. The edible oral composition can comprise one or more humectants each at a level of from 0 to about 70%, from about 5% to about 50%, from about 10% to about 60%, or from about 20% to about 80%, by weight of the edible oral composition.

Surfactants

The edible oral composition can comprise one or more surfactants. The surfactants can be used to make the compositions more cosmetically acceptable. The surfactant is preferably a detersive material which imparts to the composition detersive and foaming properties. Suitable surfactants are safe and effective amounts of anionic, cationic, nonionic, zwitterionic, amphoteric and betaine surfactants.

Suitable anionic surfactants include, for example, the water soluble salts of alkyl sulfates having from 8 to 20 carbon atoms in the alkyl radical and the water-soluble salts of sulfonated monoglycerides of fatty acids having from 8 to 20

carbon atoms. Sodium lauryl sulfate (SLS) and sodium coconut monoglyceride sulfonates are examples of anionic surfactants of this type. Other suitable anionic surfactants include sarcosinates, such as sodium lauroyl sarcosinate, taurates, sodium lauryl sulfoacetate, sodium lauroyl isethionate, sodium laureth carboxylate, and sodium dodecyl benzene sulfonate. Combinations of anionic surfactants can also be employed.

Another suitable class of anionic surfactants are alkyl phosphates. The surface active organophosphate agents can have a strong affinity for enamel surface and have sufficient surface binding propensity to desorb pellicle proteins and remain affixed to enamel surfaces. Suitable examples of organophosphate compounds include mono-, di- or triesters represented by the general structure below wherein $Z_1$, $Z_2$, or $Z_3$ may be identical or different with at least one being an organic moiety. $Z_1$, $Z_2$, or $Z_3$ can be selected from linear or branched, alkyl or alkenyl group of from 1 to 22 carbon atoms, optionally substituted by one or more phosphate groups; alkoxylated alkyl or alkenyl, (poly)saccharide, polyol or polyether group.

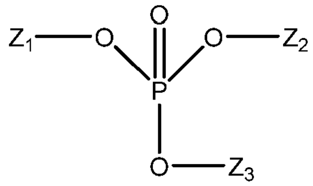

Some other agents include alkyl or alkenyl phosphate esters represented by the following structure:

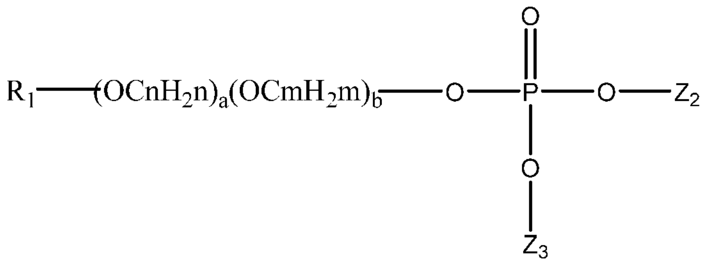

wherein $R_1$ represents a linear or branched, alkyl or alkenyl group of from 6 to 22 carbon atoms, optionally substituted by one or more phosphate groups; n and m, are individually and separately, 2 to 4, and a and b, individually and separately, are 0 to 20; Z and Z may be identical or different, each represents hydrogen, alkali metal, ammonium, protonated alkyl amine or protonated functional alkylamine, such as analkanolamine, or a R-(OCH2)(OCH) - group. Examples of suitable agents include alkyl and alkyl (poly)alkoxy phosphates such as lauryl phosphate; PPGS ceteareth-10 phosphate; laureth-1 phosphate; laureth-3 phosphate; laureth-9 phosphate; trilaureth-4 phosphate; $C_{12-18}$ PEG 9 phosphate: and sodium dilaureth-10 phosphate. The alkyl phosphate can be polymeric. Examples of polymeric alkyl phosphates include those containing repeating alkoxy groups as the polymeric portion, in particular 3 or more ethoxy, propoxy isopropoxy or butoxy groups.

Other suitable anionic surfactants are sarcosinates, isethionates and taurates, especially their alkali metal or ammonium salts. Examples include: lauroyl sarcosinate, myristoyl sarcosinate, palmitoyl sarcosinate, stearoyl sarcosinate oleoyl sarcosinate, or combinations thereof.

Other suitable anionic surfactants include sodium or potassium alkyl sulfates, such as sodium lauryl sulfate, acyl isethionates, acyl methyl isethionates, alkyl ether carboxylates, acyl alaninates, acyl gulatames, acyl glycinates, acyl sarconsinates, sodium methyl acyl taurates, sodium laureth sulfosuccinates, alpha olefin sulfonates, alkyl benze sulfonates, sodium lauroyl lactylate, sodium laurylglucosides hydroxypropyl sulfonate, and/or combinations.

Zwitterionic or amphoteric surfactants useful herein include derivatives of aliphatic quaternary ammonium, phosphonium, and Sulfonium compounds, in which the aliphatic radicals can be straight chain or branched, and one of the aliphatic substituents contains from 8 to 18 carbon atoms and one contains an anionic water-solubilizing group, e.g., carboxy, sulfonate, sulfate, phosphate or phosphonate. Suitable betaine surfactants are disclosed in U.S. Pat. No. 5,180,577. Typical alkyl dimethyl betaines include decyl betaine or 2-(N-decyl-N,N-dimethylammonio) acetate, cocobetaine or 2-(N-coco-N,N-dimethyl ammonio)acetate, myristyl betaine, palmityl betaine, lauryl betaine, cetyl betaine, cetyl betaine, stearyl betaine, *etc.* The amidobetaines can be exemplified by cocoamidoethyl betaine, cocoamidopropyl betaine (CADB), and lauramidopropyl betaine. Other suitable amphoteric surfactants include betaines, sultaines, sodium laurylamphoacetates, alkylamphodiacetates, and/or combinations thereof.

Cationic surfactants useful in the present invention include, for example, derivatives of quaternary ammonium compounds having one long alkyl chain containing from 8 to 18 carbon atoms such as lauryl trimethylammonium chloride;

cetyl pyridinium chloride; cetyl trimethylammonium bromide; cetyl pyridinium fluoride or combinations thereof.

Nonionic surfactants that can be used in the compositions of the present invention include, for example, compounds produced by the condensation of alkylene oxide groups (hydrophilic in nature) with an organic hydrophobic compound which may be aliphatic or alkylaromatic in nature. Examples of suitable nonionic surfactants can include the Pluronics® which are poloxamers, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and combinations of such materials. Other suitable non-ionic surfactants includes alkyl glucamides, alkyl glucosides, and/or combinations thereof.

The one or more surfactants can also include one or more natural and/or naturally derived surfactants. Natural surfactants can include surfactants that are derived from natural products and/or surfactants that are minimally or not processed. Natural surfactants can include hydrogenated, non-hydrogenated, or partially hydrogenated vegetable oils, olus oil, passiflora incarnata oil, candelilla cera, coco-caprylate, caprate, dicaprylyl ether, lauryl alcohol, myristyl myristate, dicaprylyl ether, caprylic acid, caprylic ester, octyl decanoate, octyl octanoate, undecane, tridecane, decyl oleate, oleic acid decylester, cetyl palmitate, stearic acid, palmitic acid, glyceryl stearate, hydrogenated, non-hydrogenated, or partially hydrogenated vegetable glycerides, Polyglyceryl-2 dipolyhydroxystearate, cetearyl alcohol, sucrose polystearate, glycerin, octadodecanol, hydrolyzed, partially hydrolyzed, or non-hydrolyzed vegetable protein, hydrolyzed, partially hydrolyzed, or non-hydrolyzed wheat protein hydrolysate, polyglyceryl-3 diisostearate, glyceryl oleate, myristyl alcohol, cetyl alcohol, sodium cetearyl sulfate, cetearyl alcohol, glyceryl laurate, capric triglyceride, coco-glycerides, lectithin, dicaprylyl ether, xanthan gum, sodium coco-sulfate, ammonium lauryl sulfate, sodium cocoyl sulfate, sodium cocoyl glutamate, polyalkylglucosides, such as decyl glucoside, cetearyl glucoside, cetyl stearyl polyglucoside, coco-glucoside, and lauryl glucoside, and/or combinations thereof. Natural surfactants can include any of the Natrue ingredients marketed by BASF, such as, for example, CegeSoft®, Cetiol®, Cutina®, Dehymuls®, Emulgade®, Emulgin®, Eutanol®, Gluadin®, Lameform®, LameSoft®, Lanette®, Monomuls®, Myritol®, Plantacare®, Plantaquat®, Platasil®, Rheocare®, Sulfopon®, Texapon®, and/or combinations thereof.

Other specific examples of surfactants include sodium lauryl sulfate, sodium lauryl isethionate, sodium lauroyl methyl isethionate, sodium cocoyl glutamate, sodium dodecyl benzene sulfonate, alkali metal or ammonium salts of lauroyl sarcosinate, myristoyl sarcosinate, palmitoyl sarcosinate, stearoyl sarcosinate and oleoyl sarcosinate, polyoxyethylene sorbitan monostearate, isostearate and laurate, sodium lauryl sulfoacetate, N-lauroyl sarcosine, the sodium, potassium, and ethanolamine salts of N-lauroyl, N-myristoyl, or N-palmitoyl sarcosine, polyethylene oxide condensates of alkyl phenols, cocoamidopropyl betaine, lauramidopropyl betaine, palmityl betaine, sodium cocoyl glutamate, and the like. Additional surfactants desired include fatty acid salts of glutamate, alkyl glucoside, salts of taurates, betaines, caprylates, and/or mixtures thereof. The edible oral composition can also be sulfate free.

The edible oral composition can comprise one or more surfactants each at a level from about 0.01% to about 15%, from about 0.3% to about 10%, or from about 0.3% to about 2.5 %, by weight of the edible oral composition.

Thickening Agents

The edible oral composition can comprise one or more thickening agents. Thickening agents can be useful in the edible oral compositions to provide a gelatinous structure that stabilizes the dentifrice and/or toothpaste against phase separation. Suitable thickening agents include polysaccharides, polymers, and/or silica thickeners.

The thickening agent can comprise one or more polysaccharides. Some non-limiting examples of polysaccharides include starch; glycerite of starch; gums such as gum karaya (sterculia gum), gum tragacanth, gum arabic, gum ghatti, gum acacia, xanthan gum, guar gum and cellulose gum; magnesium aluminum silicate (Veegum); carrageenan; sodium alginate; agar-agar; pectin; gelatin; cellulose compounds such as cellulose, microcrystalline cellulose, carboxymethyl cellulose, hydroxyethyl cellulose, hydroxypropyl cellulose, hydroxymethyl cellulose, hydroxymethyl carboxypropyl cellulose, methyl cellulose, ethyl cellulose, and sulfated cellulose; natural and synthetic clays such as hectorite clays; and mixtures thereof.

Other polysaccharides that are suitable for use herein include carageenans, gellan gum, locust bean gum, xanthan gum, carbomers, poloxamers, modified cellulose, and mixtures thereof. Carageenan is a polysaccharide derived from seaweed. There are several types of carageenan that may be distinguished by their seaweed source and/or by their degree of and position of sulfation. The thickening agent can comprise kappa carageenans, modified kappa carageenans, iota carageenans, modified iota carageenans, lambda carrageenan, and mixtures thereof. Carageenans suitable for use herein include those commercially available from the FMC Company under the series designation "Viscarin," including but not limited to Viscarin TP 329, Viscarin TP 388, and Viscarin TP 389.

The thickening agent can comprise one or more polymers. The polymer can be a polyethylene glycol (PEG), a polyvinylpyrrolidone (PVP), polyacrylic acid, a polymer derived from at least one acrylic acid monomer, a copolymer of maleic anhydride and methyl vinyl ether, a crosslinked polyacrylic acid polymer, of various weight percentages of the

edible oral composition as well as various ranges of average molecular ranges. Alternatively, the edible oral composition can be free of, essentially free of, or substantially free of a copolymer of maleic anhydride and methyl vinyl ether.

The thickening agent can comprise one or more inorganic thickening agents. Some non-limiting examples of suitable inorganic thickening agents include colloidal magnesium aluminum silicate, silica thickeners. Useful silica thickeners include, for example, include, as a non-limiting example, an amorphous precipitated silica such as ZEODENT® 165 silica. Other non-limiting silica thickeners include ZEODENT® 153, 163, and 167, and ZEOFREE® 177 and 265 silica products, all available from Evonik Corporation, and AEROSIL® fumed silicas.

The edible oral composition can comprise from 0.01% to about 15%, from 0.1% to about 10%, from about 0.2% to about 5%, or from about 0.5 % to about 2% of one or more thickening agents.

Prenylated Flavonoids

The edible oral composition of the present invention can comprise prenylated flavonoid. Flavonoids are a group of natural substances found in a wide range of fruits, vegetables, grains, bark, roots, stems, flowers, tea, and wine. Flavonoids can have a variety of beneficial effects on health, such as antioxidative, anti-inflammatory, antimutagenic, anticarcinogenic, and antibacterial benefits. Prenylated flavonoids are flavonoids that include at least one prenyl functional group (3-methylbut-2-en-1-yl, as shown in Formula VIII), which has been previously identified to facilitate attachment to cell membranes. Thus, while not wishing to being bound by theory, it is believed that the addition of a prenyl group, i.e. prenylation, to a flavonoid can increase the activity of the original flavonoid by increasing the lipophilicity of the parent molecule and improving the penetration of the prenylated molecule into the bacterial cell membrane. Increasing the lipophilicity to increase penetration into the cell membrane can be a double-edged sword because the prenylated flavonoid will tend towards insolubility at high Log P values (high lipophilicity). Log P can be an important indicator of antibacterial efficacy.

As such, the term prenylated flavonoids can include flavonoids found naturally with one or more prenyl functional groups, flavonoids with a synthetically added prenyl functional group, and/or prenylated flavonoids with additional prenyl functional groups synthetically added.

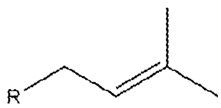

Formula VIII. Prenyl Function Group with R representing the other portions of the molecule

Other suitable functionalities of the parent molecule that improve the structure-activity relationship (e.g,. structure-MIC relationship) of the prenylated molecule include additional heterocycles containing nitrogen or oxygen, alkylamino chains, or alkyl chains substituted onto one or more of the aromatic rings of the parent flavonoid.

Flavonoids can have a 15-carbon skeleton with at least two phenyl rings and at least one heterocyclic ring. Some suitable flavonoid backbones can be shown in Formula IX (flavone backbone), Formula X (isoflavan backbone), and/or Formula XI (neoflavonoid backbone).

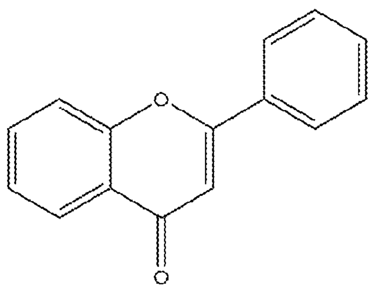

Formula IX. Flavone Backbone

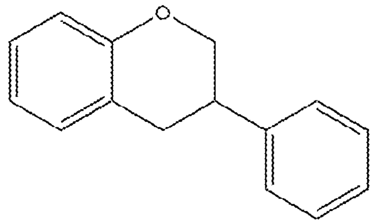

Formula X. Isoflavan backbone

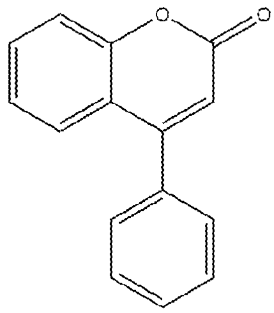

Formula XI. Neoflavanoid backbone

Other suitable subgroups of flavonoids include anthocyanidins, anthoxanthins, flavanones, flavanonols, flavans, isoflavonoids, chalcones and/or combinations thereof.

Prenylated flavonoids can include naturally isolated prenylated flavonoids or naturally isolated flavonoids that are synthetically altered to add one or more prenyl functional groups through a variety of synthetic processes that would be known to a person of ordinary skill in the art of synthetic organic chemistry.

Other suitable prenylated flavonoids can include Bavachalcone, Bavachin, Bavachinin, Corylifol A, Epimedin A, Epimedin A1, Epimedin B, Epimedin C, Icariin, Icariside I, Icariside II, Icaritin, Isobavachalcone, Isoxanthohumol, Neobavaisoflavone, 6-Prenylnaringenin, 8-Prenylnaringenin, Sophoraflavanone G, (-)-Sophoranone, Xanthohumol, Quercetin, Macelignan, Kuraridin, Kurarinone, Kuwanon G, Kuwanon C, Panduratin A, 6-geranylnaringenin, Australone A, 6,8-Diprenyleriodictyol, dorsmanin C, dorsmanin F, 8-Prenylkaempferol, 7-O-Methylluteone, luteone, 6-prenylgenistein, isowighteone, lupiwighteone, and/or combinations thereof. Other suitable prenylated flavonoids include cannflavins, such as Cannflavin A, Cannflavin B, and/or Cannflavin C.

Preferably, the prenylated flavonoid has a high probability of having an MIC of less than about 25 ppm for *S. aureus,* a gram-positive bacterium. Suitable prenylated flavonoids include Bavachin, Bavachinin, Corylifol A, Icaritin, Isoxanthohumol, Neobavaisoflavone, 6-Prenylnaringenin, 8-Prenylnaringenin, Sophoraflavanone G, (-)-Sophoranone, Kurarinone, Kuwanon C, Panduratin A, and/or combinations thereof.

Preferably, the prenylated flavonoid has a high probability of having an MIC of less than about 25 ppm for *E. coli,* a gram-negative bacterium. Suitable prenylated flavonoids include Bavachinin, Isoxanthohumol, 8-Prenylnaringenin, Sophoraflavanone G, Kurarinone, Panduratin A, and/or combinations thereof.

Approximately 1000 prenylated flavonoids have been identified from plants. According to the number of prenylated flavonoids reported before, prenylated flavonones is the most common subclass and prenylated flavanols is the rarest sub-class. Even though natural prenylated flavonoids have been detected to have diversely structural characteristics, they have a narrow distribution in plants, which are different to the parent flavonoids as they are present almost in all plants. Most of prenylated flavonoids are found in the following families, including *Cannabaceae, Guttiferae, Leguminosae, Moraceae, Rutaceae* and *Umbelliferae. Leguminosae* and *Moraceae,* due to their consumption as fruits and vegetables, are the most frequently investigated families and many novel prenylated flavonoids have been explored. *Humulus lupulus* of the *Cannabaceae* include 8-prenylnaringenin and xanthohumol, which play an important role in the health benefits of beer.

The prenylated flavonoid can be incorporated through the hops extract, incorporated in a separately added extract, or added as a separate component of the edible oral compositions disclosed herein.

## Vitamin

The edible composition can comprise one or more vitamins. As used herein, "vitamin" includes all natural and/or synthetic analogs of vitamins, vitamers, compounds and/or derivatives that exhibit the biologically activity of vitamins,

isomers of these compounds, stereoisomers of these compounds, salts of these compounds, or combinations thereof.

Suitable vitamins for gum health can include Vitamin A, such as retinoid compound, Vitamin B, including Vitamin B1 (thiamine), Vitamin B2 (riboflavin), Vitamin B3 (niacin), Vitamin B5 (pantothenic acid), Vitamin B6, Vitamin B7 (biotin), Vitamin B9 (folic acid and/or folate), Vitamin B12 (cyancobalamin), Vitamin C, Vitamin D, Vitamin E, Vitamin K, and/or combinations thereof. Vitamins can also include other vitamin-like compounds, such as choline, carnitine, or combinations thereof.

The oral care composition can comprise from about 0.0001% to about 10%, from about 0.01% to about 5%, or from about 0.01% to about 2%, by weight of the composition, of vitamin.

## Retinoid Compound

The compositions of the present invention can comprise one or more retinoid compounds. As used herein, "retinoid compound" includes all natural and/or synthetic analogs of Vitamin A or retinol-like compounds that possess the biological activity of Vitamin A in the skin as well as the geometric isomers and stereoisomers of these compounds. The retinoid compound can, for example, be retinol, retinyl esters (e.g., C-C alkyl esters of retinol, including retinyl palmitate, retinyl acetate, retinyl propionate), retinal, and/or retinoic acid (including all-trans retinoic acid and/or 13-cis-retinoic acid). In some embodiments, retinoids other than retinoic acid are used. These compounds are available in the art and are commercially available from several sources, e.g., Sigma Chemical Company (St. Louis, Mo.), and Boerhinger Mannheim (Indianapolis, Ind.). Other suitable retinoids are tocopheryl-retinoate, tocopherol ester of cis- or trans-retinoic acid, adapalene (6-3-(1-adamantyl)- 4-methoxyphenyl-2-naphthoic acid), and tazarotene (ethyl 6-2-(4.4-dimethylthiochroman-6-yl)-ethynylnicotinate). Desirable retinoids include retinol, retinoic acid, retinyl palmitate, retinyl acetate, retinyl propionate, retinal, and combinations thereof.

The retinoid compound may be included as the substantially pure material, or as an extract obtained by suitable physical and/or chemical isolation from natural (e.g., plant) sources. The retinoid compound can be substantially pure, or essentially pure. The compositions of this invention may contain a safe and effective amount of the retinoid compound, such that the oral care composition is safe and effective for regulating or improving the condition of keratinous tissues and accidental ingestion since applied to the oral cavity.

The retinoid compound can comprise retinol, retinyl ester, retinal, retinoic acid, tocopheryl-retinoate, tocopherol ester of *cis-* or *trans-retinoic* acid, isotretinoin, alitretinoin, etretinate, acitretin, adapalene, bexarotene, tazarotene, or combinations thereof. The retinoid compound can be pharmaceutical grade, USP, or the like grade, due to use in the oral cavity. The retinoid compound and/or the retinol can have a purity of at least about 95%, at least about 97%, at least about 99%, at least about 99.5%, or at least about 99.9%. The oral care composition can comprise from about 0.0001% to about 10%, from about 0.01% to about 5%, or from about 0.01% to about 2%, by weight of the composition, of retinoid compound.

The retinoid compound can comprise retinol comprising *cis-* and/or *trans-* alkene functional groups. The retinol can comprise at least about 80%, at least about 90%, and/or at least about 95% *trans*-alkene functional groups.

The retinoid compound can also comprise surfactant, such as anionic surfactant, cationic surfactant, and/or nonionic surfactant, which can improve gum barrier permeability. Suitable surfactants can include polysorbate.

## Amino Acid

The edible oral composition can comprise amino acid. The amino acid can comprise one or more amino acids, peptide, and/or polypeptide, as described herein. Unexpectedly, it has been found that the combination of retinoid compound and amino acid can improve the gum health of a user.

Amino acids, as in Formula XII, are organic compounds that contain an amine functional group, a carboxyl functional group, and a side chain (R in Formula XII) specific to each amino acid. Suitable amino acids include, for example, amino acids with a positive or negative side chain, amino acids with an acidic or basic side chain, amino acids with polar uncharged side chains, amino acids with hydrophobic side chains, and/or combinations thereof. Suitable amino acids also include, for example, arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutonic acid, diaminoproprionic acid, salts thereof, and/or combinations thereof.

Suitable amino acids include the compounds described by Formula XII, either naturally occurring or synthetically derived. The amino acid can be zwitterionic, neutral, positively charged, or negatively charged based on the R group and the environment. The charge of the amino acid would be well known to one of ordinary skill in the art.

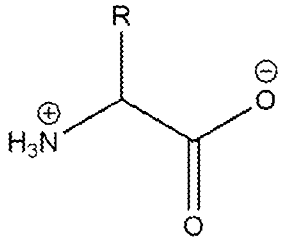

Formula XII. Amino Acid. R is any suitable functional group

Suitable amino acids include one or more basic amino acids, one or more acidic amino acids, one or more neutral amino acids, or combinations thereof.

The edible oral composition can comprise from about 0.01% to about 20%, from about 0.1% to about 10%, from about 0.5% to about 6%, or from about 1% to about 10 % of amino acid, by weight of the oral care composition.

The term "neutral amino acids" as used herein include not only naturally occurring neutral amino acids, such as alanine, asparagine, cysteine, glutamine, glycine, isoleucine, leucine, methionine, phenylalanine, proline, serine, threonine, tryptophan, tyrosine, valine, but also biologically acceptable amino acid which has an isoelectric point in range of pH 5.0 to 7.0. The biologically preferred acceptable neutral amino acid has a single amino group and carboxyl group in the molecule or a functional derivative hereof, such as functional derivatives having an altered side chain albeit similar or substantially similar physio chemical properties. In a further embodiment the amino acid would be at minimum partially water soluble and provide a pH of less than 7 in an aqueous solution of 1 g/1000 mL at 25°C.

Accordingly, neutral amino acids suitable for use in the invention include, but are not limited to, alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, salts thereof, or mixtures thereof. Preferably, neutral amino acids used in the composition of the present invention may include asparagine, glutamine, glycine, salts thereof, or mixtures thereof. The neutral amino acids may have an isoelectric point of 5.0, or 5.1, or 5.2, or 5.3, or 5.4, or 5.5, or 5.6, or 5.7, or 5.8, or 5.9, or 6.0, or 6.1, or 6.2, or 6.3, or 6.4, or 6.5, or 6.6, or 6.7, or 6.8, or 6.9, or 7.0, in an aqueous solution at 25°C. Preferably, the neutral amino acid is selected from proline, glutamine, or glycine, more preferably in its free form (i.e. uncomplexed). If the neutral amino acid is in its salt form, suitable salts include salts known in the art to be pharmaceutically acceptable salts considered to be physiologically acceptable in the amounts and concentrations provided. Preferably the neutral amino acid is present in the amount of from about 0.0001% to about 10%, preferably from about 0.05% to about 5%, preferably from about 0.1% to about 3%, preferably from about 0.5% to about 3%, preferably from about 1% to about 3%, by weight of the composition. In one aspect, the neutral amino acid is glutamine (or salt thereof). In another aspect, the neutral amino acid is proline (or salt thereof). In yet another aspect, the neutral amino acid is glycine (or salt thereof).

The edible oral composition can comprise from about 0.0001% to about 20%, from about 0.1% to about 10%, from about 0.5% to about 6%, or from about 1% to about 10 % of neutral amino acid, by weight of the oral care composition.

Peptide

The edible oral composition can also comprise a peptide. A peptide is a linear organic polymer consisting of a number of amino-acid residues bonded together in a chain, forming part of (or the whole of) a protein molecule. The peptide can comprise from two amino acids to ten amino acids, from two amino acids to five amino acids, or from four amino acids to six amino acids.

Peptides, including but not limited to, di-, tri-, tetra-, and pentapeptides and derivatives thereof, may be included in the compositions of the present invention in amounts that are safe and effective, including safe and effective for ingestion. As used herein, "peptides' refers to both the naturally occurring peptides and synthesized peptides. Also, useful herein are naturally occurring and commercially available compositions that contain peptides.

Suitable dipeptides for use herein include, for example, Carnosine (beta-ala-his). Suitable tripeptides for use herein include, for example, gly-his-lys, arg-lys-arg, and/or his-gly-gly. Suitable tripeptide derivatives include palmitoyl-gly-his-lys, which may be purchased as Biopeptide CLTM (100 ppm of palmitoyl-gly his-lys commercially available from Sederma, France); Peptide CK (arg-lys-arg); Peptide CK(ac-arg-lys-arg-$NH_2$); and a copper derivative of his-gly-gly sold commercially as lamin, from Sigma (St. Louis, Mo.). Suitable tetrapeptides for use herein include, for example, Peptide E, arg-ser-arg-lys.

Suitable pentapeptides for use herein include lys-thr-thr-lys-ser. A preferred commercially available pentapeptide derivative composition is MatrixylTM, which contains 100 ppm palmitoyl-lys-thr-thr-lys-ser (commercially available from Sederma France).

The peptide can comprise palmitoyl-lys-thr-thr lys-ser, palmitoyl-gly-his-lys, beta-ala-his, their derivatives, and/or combinations thereof. In some embodiments, the peptide comprises palmitoyl-lys-thr-thr-lys-ser, palmitoyl-gly-his-lys, their derivatives, or combinations thereof. In other embodiments, the peptide comprises palmitoyl-lys-thr-thr-lys-ser (pal-KTTKS) and/or derivatives thereof. Other suitable peptides include gly-his-ly (GHK), gly-glu-lys-gly (GEKG), or combinations thereof.

The edible oral composition can comprise from about 0.0001% to about 10%, from about 0.01% to about 5%, from about 0.001% to about 5%, from about 0.01% to about 2%, or from about 0.0001% to about 1%, by weight of the composition of peptide.

Other Ingredients

The edible oral composition can comprise a variety of other ingredients, such as flavoring agents, sweeteners, colorants, preservatives, buffering agents, or other ingredients suitable for use in edible oral compositions, as described below.

Flavoring agents also can be added to the edible oral composition. Suitable flavoring agents include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-iris one, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants in the present compositions are the paramenthan carboxyamide agents such as N-ethyl-p-menthan-3-carboxamide (known commercially as "WS-3") or N-(Ethoxycarbonylmethyl)-3-p-menthanecarboxamide (known commercially as "WS-5"), and mixtures thereof. A flavor system is generally used in the compositions at levels of from about 0.001 % to about 5%, by weight of the edible oral composition. These flavoring agents generally comprise mixtures of aldehydes, ketones, esters, phenols, acids, and aliphatic, aromatic and other alcohols.

Sweeteners can be added to the edible oral composition to impart a pleasing taste to the product. Suitable sweeteners include saccharin (as sodium, potassium or calcium saccharin), cyclamate (as a sodium, potassium or calcium salt), acesulfame-K, thaumatin, neohesperidin dihydrochalcone, ammoniated glycyrrhizin, dextrose, levulose, sucrose, mannose, sucralose, stevia, and glucose.

Colorants can be added to improve the aesthetic appearance of the product. Suitable colorants include without limitation those colorants approved by appropriate regulatory bodies such as the FDA and those listed in the European Food and Pharmaceutical Directives and include pigments, such as $TiO_2$, and colors such as FD&C and D&C dyes.

Preservatives also can be added to the edible oral compositions to prevent bacterial growth. Suitable preservatives approved for use in oral compositions such as methylparaben, propylparaben, benzoic acid, and sodium benzoate can be added in safe and effective amounts.

Titanium dioxide may also be added to the present composition. Titanium dioxide is a white powder which adds opacity to the compositions. Titanium dioxide generally comprises from about 0.25% to about 5%, by weight of the edible oral composition.

Other ingredients can be used in the edible oral composition, such as desensitizing agents, healing agents, other caries preventative agents, chelating/sequestering agents, , proteins, other anti-plaque/anti-calculus agents, opacifiers, antibiotics, anti-enzymes, enzymes, pH control agents, oxidizing agents, antioxidants, and the like.

Forms

The edible oral composition can be provided in many forms. Suitable forms include gummy composition, a chewable composition, a pan-chew composition, a tablet composition, a dissolving tablet composition, a dissolving film, a leave-on composition, a lollipop composition, a lozenge composition, a nonwoven fiber composition, a soluble foam composition, a liquid composition, a paste composition, chewing gum composition, or combinations thereof.

The edible oral composition can be a gummy composition and/or soft chewable composition, such as described in United States Patent Application Publication No. 2017/0360865.

The edible oral composition can be a leave-on composition. Suitable leave-on compositions include dispersions of hops in hydrophobic phases, such as petrolatum as described in U.S. Patent No. 10 849,729, emulsions, as described in U.S. Patent Application Publication No. 2018/013319, jammed inverse emulsion, such as described in U.S. Patent No. 10,780,032, and hydrophilic leave-on compositions.

The leave-on composition can be applied to the teeth, and left on for more than 2 minutes, preferably more than 10 minutes, more preferably more than 30 minutes and more preferably 60 minutes or longer. Preferably, the leave-on composition is applied to the teeth as the last step of oral hygiene regimen. For example, the leave-on composition of the present invention is applied after brushing teeth, and optionally after using mouth rinse and/or floss.

In one aspect, the present invention is directed to an edible oral composition which is in a gel form. It is desirable to

have a gel for use in the present invention that enables easy application, thin layer formation and evenly spread into gingival sulcus/pockets and along gingival gum line. The oral care composition has a Viscosity Consistency Coefficient K of about 20 Pa·s to about 500 Pa·s, as measured by the Rheological Test method described herein. Preferably, the oral care composition has a Viscosity Consistency Coefficient K of about 20 Pa·s to about 500 Pa·s, preferably from about 30 Pa·s to about 400 Pa·s, more preferably from about 40 Pa·s to about 300 Pa·s, even more preferably from 50 Pa·s to 250 Pa·s. This optimal viscosity profile range provides better sensory experience of spread ability for a user. If a product is too viscous, it would be hard for a user to spread it evenly onto gingival tissue. If the product has a too low viscosity, it is runny and hard to be retained on appropriate area by finger or applicator. Alternatively, the edible oral composition can be applied in retained within the use of mouth tray/guard.

In one aspect, the edible composition of present invention has a desirable mucoadhesion property. Mucoadhesion can be defined as adhesive interaction between two surfaces where one is at least mucosa for a given period through interfacial forces with a consequent decreased in the surface energy. Mucoadhesion polymers for oral care application should ideally (1) easily retain hydrophilic and lipophilic active ingredients and not hinder their release; (2) promote active ingredient penetration and absorption, (3) adhere as quickly as possible to biological substrate and be retained for a period of time, (4) be safe, (5) be cost effective and (6) provide user acceptable application.

The edible oral of present invention has a Mucoadhesion Index in the range of not less than 0.3 FI%, as measured by the Mucoadhesion Test Method described herein. Preferably, the oral care composition has a Mucoadhesion Index of no less than about 0.5 FI%, and more preferably no less than about 1.0 FI%.

The edible oral composition can be a soluble fiber composition and/or a soluble foam composition as described in U.S. Patent No. 10,932 996.

Methods of Use

The references to methods of treatment in this description are to be interpreted as references to the compounds, pharmaceutical compositions and medicaments of the present invention for use in a method for treatment of the human (or animal) body by therapy (or for diagnosis). They are not part of the invention.

The edible compositions of the present invention can be used in the treatment, reduction, and/or prevention of caries, cavities, gingivitis, and/or combinations thereof.

The edible oral compositions useful for the methods include hops, as described above, such as hops provided from a hops extract and/or an extract of *Humulus lupulus*. As described herein hops beta acid can be useful as an anticavity agent. Thus, the addition of hops to any edible composition can provide anticavity protection as long as the edible oral composition can have sufficient contact time with the targeted teeth.

The edible oral composition can include primary packaging, such as a tube, bottle, and/or tub. The primary package can be placed within secondary package, such as a carton, shrink wrap, or the like. Instructions for use of the oral care composition can be printed on the primary package and/or the secondary package. The scope of the method is intended to include instructions provided by a manufacturer, distributor, and/or producer of the oral care composition.

The method of use for each edible oral composition can differ based on the form. For example, a soft chewable can be chewed by a user after eating, drinking or at virtually any other time of the day for the treatment, reduction, and/or prevention of caries, cavities, gingivitis, and/or combinations thereof.

If the edible oral composition is a lollipop, it can placed in the oral cavity of user after eating, drinking or at virtually any other time of the day for the treatment, reduction, and/or prevention of caries, cavities, gingivitis, and/or combinations thereof.

If the edible oral composition is a gum, the gum can be chewed by a user after eating, drinking or at virtually any other time of the day for the treatment, reduction, and/or prevention of caries, cavities, gingivitis, and/or combinations thereof.

Each of the edible oral composition can be swallowed after a user is complete with the application, but the edible oral composition does not have to be swallowed for the oral health benefit.

Method of Making a Soft Chewable Composition

The present disclosure also relates to processes, not part of the invention, for making a soft chewable composition containing hops.

In one example, a method of preparing a soft chewable composition, wherein the soft chewable composition is a gummy, can comprise the steps of:

a. adding a binding agent to a first mixing vessel;
b. pre-treating the binding agent;
c. adding a humectant component and water to a second mixing vessel and mixing while heating to a temperature of

from about 65°C to about 72°C;

d. adding a polyol to the second mixing vessel to form a syrup pre-mixture and mixing while heating to form a cooked syrup pre-mixture;

e. adding the pre-treated binding agent to the second vessel and mixing while heating to form a base syrup mixture;

f. adding a processing aid to the base syrup mixture and mixing while heating;

g. adding a hops mixture to the base syrup mixture and mixing to form a final mixture;

h. optionally heating the final mixture to a temperature sufficient to achieve a desired solids content;

i. forming the final mixture into a soft chewable composition by molding;

j. cooling and optionally curing the soft chewable composition; and

k. optionally post-processing the soft chewable composition.

The syrup pre-mixture can be heated to a temperature of about 93°C to about 177°C. The syrup pre-mixture can be heated to a temperature of about 113°C.

The pre-treatment step can vary depending on the binding agent used in the formula. In the case of a gelatin binding agent, pre-treating can comprise of hydrating the gelatin by adding water to the gelatin at a ratio of about 2:1 to about 3:1 and mixing at room temperature until the gelatin is completely hydrated. In the case of a starch binding agent, pre-treating can comprise of gelatinizing the starch by adding water to the starch and heating while mixing to a temperature of about 77°C until the color of the starch binding agent changes from opaque white to clear grey. In the case of a pectin binding agent, pre-treating can comprise of mixing sucrose with the pectin to create a pectin-sucrose mix.

Additional ingredients, such as coloring agents, flavoring agents, processing aids, salts, food grade acids, supplement components, active ingredients, and combinations thereof, can be added to the cooked syrup pre-mixture. One advantage to adding the additional ingredients to the cooked syrup pre-mixture is that these ingredients may be temperature sensitive. The additional ingredients can be added to the base syrup mixture. Alternatively, the additional ingredients can be added to the base syrup mixture after adding the processing aid. Alternatively, the additional ingredients can be added to the final mixture. One advantage to adding the additional ingredients to the final mixture is that it can help to delay hydration and/or aid in processability.

The processing aid can first be heated in a separate mixing vessel to a temperature above its melting point before it is added to the base syrup mixture. The processing aid can be shortening and can be heated to a temperature greater than about 47°C. Alternatively, the processing aid can be separately melted before it is added to the base syrup mixture.

A hops mixture can comprise hops. Alternatively, a hops mixture can comprise hops and additional ingredients. A hops mixture can be prepared by mixing hops with the additional ingredients before it is mixed with the processing aid. A hops mixture can be formed by combining hops, citric acid, a flavoring agent, and a coloring agent. Alternatively, the hops mixture can be mixed with the processing aid before it is added to the base syrup mixture. A salt can also be added to the hops mixture before it is added to the base syrup mixture.

The hops mixture can be added to the base syrup mixture just prior to molding to prevent a significant increase in viscosity. It was found that increasing the temperature of hops up to about 95°C significantly reduces the viscosity. However, this increase in temperature can also increase the hydration rate of hops particles, resulting in an increased viscosity again after 15 minutes. The final mixture can be processed in a mold or extruded within about 15 minutes of adding hops. Adding citric acid and/or salt to the hops mixture before it is added to the base syrup mixture can help to reduce the viscosity of the syrup and can increase the time for molding and/or extrusion to about 20 minutes, alternatively about 30 minutes, alternatively about 60 minutes, alternatively about 90 minutes.

The final mixture can be mixed for about 5 minutes to about 60 minutes, alternatively for about 10 minutes to about 50 minutes, alternatively for about 15 minutes to about 40 minutes, alternatively for about 20 minutes to about 30 minutes. One advantage to mixing the final mixture is that it can reduce the viscosity of the final mixture and increase the time for molding and/or extrusion. After the final mixture has started gelling, physical sheer, such as mixing or pumping, can be used to break up the gel structure and lower the viscosity.

The hops can be agglomerated with an agglomerating material. The agglomerating materials useful herein are known, having been described in detail in U.S. Pat. No. 5,340,580 to Barbera, and U.S. Pat. Nos. 4,548,806 and 4,459,280, both to Colliopoulos et al. These agglomerating materials are selected from the group consisting of water dispersible hydrolyzed starch oligosaccharide, monosaccharide, di-saccharide, polyglucose, polymaltose, and mixtures thereof. The agglomerating material can include sucrose, salt, acid, maltodextrin, and combinations thereof. The soft chewable composition can comprise from about 0.5% to about 20% of agglomerating material coating on the hops, alternatively from about 1% to about 10%, alternatively from about 1% to about 5%.

The hops can be agglomerated before it is added to the base syrup mixture. The agglomeration process can comprise the steps of (a) coating to agglomerate a hops-containing blend, preferably a dry blend, with a solution mixture comprising one or more agglomerating materials; (b) drying the agglomerated hops; and (c) optionally, repeating steps (a) and (b). Step (c) is only optional, however, if one coating and drying step is sufficient to uniformly disperse at least about 0.5% of the acid throughout the agglomerating material coating on the hops, otherwise it is necessary to repeat steps (a)

and (b) at least as many times as necessary to attain at least this level of acid uniformly dispersed.

Agglomeration techniques are described in the hereinbefore referenced U.S. patents. In one example, a multiple layer coating is applied to the hops using techniques which result in agglomerating the hops, e.g., as described in detail in U.S. Pat. Nos. 4,459,280 and 4,548,806, to Colliopoulos et al., is used. In another example, an agglomerating material (especially maltodextrin) is applied as a single coating in a single pass apparatus such that from about 5% to about 20% of water is applied to the hops during the coating process is used.

Multiple layer coating of the hops is accomplished, for example, by using fluid bed agglomerating equipment. An example of such fluid bed agglomerating equipment is the Fluid Air, Inc., Model 0300 Granulator-Dryer (sold by Fluid Air, Inc., Aurora, Illinois). Single layer coating of the hops is achieved by utilizing equipment which operates preferably by dropping a dry blend hops-containing material through a highly turbulent annular zone formed by a cylindrical wall and a rotating shaft with variously pitched attached blades. An agglomerating material-containing solution, is sprayed into this zone to contact the dry hops-containing blend. The resulting coating hops is dropped to a fluid bed dryer where the added solvent is removed. An example of this equipment is the Bepex Turboflex Model No. TFX-4 (sold by Bepex Corporation; Minneapolis, Minn.) with a six square foot bed vibrating fluid bed dryer (sold by Witte Corporation, Inc., Washington, N.J.).

The hops can be blended with about 70% sucrose and then sprayed with a 40% solution of citric acid followed by fluid bed drying.

One advantage to agglomerating the hops before adding it to the base syrup mixture is that it can help to delay the hydration of hops and can help to lower viscosity. Another advantage is that it can help improve the mouthfeel of the soft chewable composition. It is believed that the use of agglomerated hops can increase the dissolution rate of hops in the mouth, thereby reducing mouth dryness, and can reduce the gelling of hops in the mouth.

Alternatively, the hops can be unagglomerated. The humectant, polyol, and water can be combined in the second mixing vessel and heated to a temperature of about 113°C to form a cooked syrup pre-mixture. The pre-treated binding agent can then be added to the cooked syrup pre-mixture to form the base syrup mixture.

The final mixture can be formed into a soft chewable composition by molding or extrusion. The final mixture can be poured into a starch mold, via the Mogul process, or in a non-absorbent mold to create a soft chewable composition. Non-limiting examples of non-absorbent molds can include polymer, glass, metal, plastic, polytetrafluoroethylene, and any other material that does not absorb moisture.

The final mixture can be poured into a starch mold and allowed to cure. The starch mold can be any shape that is created by printing on the surface of the starch using a metallic board. The final mixture can be poured into a sheet mold to create a sheet of the soft chewable composition. The sheet can be cut into individual pieces and placed into starch molds to cure. The individual cut pieces can be placed in a tumbling drum with starch and continuously mixed for the time needed to increase the solids content to about 70 to about 80%. In one example, the soft chewable composition can cure for about 1 day to about 5 days before packaging. The soft chewable composition can cure for about 4 days, in another example for about 2 days, and in another example about 1 day before packaging. The curing time can be reduced by filling the mold with a final mixture that is at the target solids content. The soft chewable composition can be cured at room temperature, alternatively the soft chewable composition can be cured at about 22°C to about 60°C. The soft chewable composition can be cured in a curing room with about 15% to about 25% RH.

Alternatively, the final mixture can be poured into a non-absorbent mold and allowed to cool. When using a non-absorbent mold, the solids concentration of the final mixture can be close to the desired finished product solids level because no significant changes in moisture will occur. The non-absorbent mold can provide the shape of the soft chewable composition, alternatively the soft chewable composition can be cut into the desired shape after it is removed from the non-absorbent mold. The soft chewable composition can be placed in a refrigerator and cooled to a temperature of about 20°C to about 40°C.

The soft chewable composition can optionally be post-processed to decrease curing time, control texture such as adhesiveness, improve taste, improve stability, improve processability, and/or facilitate the dosing of the hops. Post-processing can include cutting, drying, individually wrapping the soft chewable composition pieces, dusting the soft chewable composition with sugar or starch after removal from the mold, coating the soft chewable composition after removal from the mold, leaving the soft chewable composition in the mold until the desired adhesiveness is achieved, enrobing, coextrusion, and combinations thereof. One advantage to post-processing the soft chewable composition is that it can prevent individual pieces of the soft chewable composition from sticking together during packaging and can make them feel less sticky during handling. Another advantage to post-processing is that it can reduce viscosity and increase time for molding and/or extrusion which can improve processability.

In one example, a method of preparing a low water soft chewable composition can comprise the steps of:

a. adding a humectant component to a first mixing vessel;
b. mixing while heating the first mixing vessel to a temperature of from about 65°C to about 71°C;
c. adding polyol to the first mixing vessel to form a humectant-syrup pre-mix and mixing while heating to form a cooked humectant-syrup mixture;

d. mixing a processing aid and a hops mixture in a third mixing vessel to form a hops-processing aid mixture;
e. adding the hops-processing aid mixture to the cooked humectant-syrup mixture and mixing while heating to form the final dough;
f. forming the final dough into the soft chewable composition with extrusion dies or wire cutting; and
g. optionally post-processing the soft chewable composition.

The hops mixture can be added to the cooked humectant-syrup mixture to form a final dough and extruded to form the soft chewable composition in the desired shape and size. Alternatively, the final dough can be spread into a tray and cut into pieces. The soft chewable composition can be cooled to a temperature of about 20°C to about 40°C before packaging.

It should be understood that the formulation for the soft chewable composition can be designed to achieve a specific final solids content without the need for heat to temperatures above the boiling point to evaporate solids using high levels of plasticizers such as oils and emulsifiers. Alternatively, the formulation for the soft chewable composition can be designed such that boiling is required during processing to achieve the desired solids content.

The soft chewable composition can be formed into any suitable convenient, ingestible form. Non-limiting examples of the form of the compositions include: soft chew, hard chew, soft gel, semi-solid taffy-like chew, gummies, and combinations thereof. The soft chewable composition can be in the form of a single piece of soft chew or a single piece of gummy. The soft chewable composition can be in a partitionable form, such as a bar, which the user can cut or break to provide individual pieces. A piece of the soft chewable composition can be from about 500 to about 7000 $mm^3$, alternatively from about 1000 to about 5000 $mm^3$, alternatively from about 1500 to about 4000 $mm^3$. A piece of the soft chewable composition can have a volume of about 100,000 $mm^3$ and can be broken into smaller pieces. The soft chewable composition can be formed into any shape and size as long as it provides a volume within this range. Non-limiting examples of shapes can include circles, squares, rectangles, stars, hearts, animal shapes, and combinations thereof.

The soft chewable compositions can be packaged in any suitable package. The soft chewable composition can be individually wrapped in food grade packaging. The soft chewable composition can be individually wrapped and packaged together with enough pieces for a single dose, alternatively enough for a daily dose. Non-limiting examples of food grade packaging can include monoaxially oriented polypropylene, poly-lined foil wrappers, foil, and combinations thereof. Alternatively, the soft chewable composition can be unwrapped.

The soft chewable composition can be placed in secondary packaging, non-limiting examples of which include glass bottles; plastic bottles; foil lined bags, foil lined containers, cartons, or sleeves; and combinations thereof. The soft chewable composition can be packaged as single doses so they are easily portable and can be carried in a purse, pocket, or brief case. The packaging can be child resistant. The packaging can be transparent, alternatively the packaging can be opaque. The package can include a desiccant. The secondary packaging can contain an ultraviolet (UV) inhibitor because the soft chewable composition can be light sensitive. Alternatively, the secondary packaging does not contain a UV-inhibitor. The secondary packaging can contain a water and/or oxygen barrier because the soft chewable composition can be water and/or oxygen sensitive. Alternatively, the secondary packaging does not contain a water and/or oxygen barrier.

## EXAMPLES

The invention is further illustrated by the following examples, the invention is defined by the claims. Any subject-matter falling outside the scope of the claims is provided for information purposes only.

### Experimental Methods

#### Acid Production and Acid Inhibition [%]

Acid production and acid inhibition were determined with the in vitro plaque glycolysis model (iPGRM). The purpose of this technique is to provide a simple and quick method for determining if compounds have an influence on the metabolic pathways that plaque microorganisms utilize to produce toxins that adversely affect gingival health. It is also useful to determine if compounds have an influence on the metabolic pathways that plaque microorganisms utilize to produce plaque acids that adversely affect teeth.

The *in vitro* plaque glycolysis model (iPGRM) is a technique in which plaque is grown from human saliva and treated with various agents to determine anti-glycolytic activity of treatments. When bacteria convert sugar into energy with the help of enzymes, acids are formed. These acids demineralize and damage the dental enamel. The purpose of this technique is to provide a simple and quick method for determining if treatment compounds have an inhibitory effect on the metabolic pathways that plaque microorganisms utilize for the production of acids or toxins and/or inhibit their growth. For the purposes of the work here, if the test therapeutic compositions contain Sn, the Sn placebo should be tested. Additionally, the antibacterial composition should be tested with respect to its placebo to determine the iPGRM value for the antibacterial composition only. This can be important if buffers, e.g., bicarbonate, orthophosphate, calcium

carbonate, are present in the composition in addition to the antibacterial composition.

A plaque biofilm was grown on glass rods from fresh pooled human saliva and Trypticase Soy Broth (TSB) at 37 °C over 2 days by dipping glass rods into and out of media in a reciprocating motion. Treatments were 2 minutes of dentifrice slurry in water (1:5) or diluted treatment in water (1:5). The edible compositions of TABLE 1 were prepared for the iPGRM test by combining 4g of edible composition with 20g of ultra-pure water and stirring overnight before the treatment of the 3-day biofilm. The edible compositions of TABLE 1 comprising hops were prepared by combining 4g of edible composition with 16g of ultra-pure water and stirring overnight then by adding 4g of a 0.5% solution of Hops Beta Acids extract in water the morning of the treatment of the 3-day biofilm. The 0.5% Hops Beta Acids extract in ultra-pure water was made by combining 1.11g of Hops Beta Acids extract with 98.89g of ultra-pure water the morning of the treatment of the 3-day biofilm. All treatments that did not completely dissolve overnight were homogenized briefly to ensure uniformity using a high shear mixer. Prior to treatments, the pH of each dissolved/homogenized sample was obtained and was adjusted to pH 7 immediately prior to treatment.

After treatments, biofilms were incubated with TSB and sucrose until pH indicator showed a color change (~ 6hrs). The pH of the media solutions was then measured to determine the amount of glycolysis inhibition relative to a negative control.

On Day 1, new glass rods (5 mm x 90 mm) were polished approximately 25 mm from the un-tapered end on a lathe with silicon carbide paper of 240, 320, 400, and 600 grit used sequentially. After the initial polishing, the rods should be polished with 600 grit paper before each test. After polishing, rods were stored until ready to run test. Enough rods should be polished for a full rack of treatments. A rack can treat 12 compositions with 4 replicates of each composition such that the rack has 48 rods.

On Day 2, saliva was collected daily during the test from a panel of 5 - 10 people by paraffin stimulation and was refrigerated at 4°C until it was needed throughout the day. Pool saliva carefully (do not pour in wax/mucus) and mix thoroughly before use. The rods were removed from storage, rinsed with deionized water to remove any sanding residue, disinfected in 70% ethanol/water solution, and were allowed to dry on a sterile surface. Subsequently, the rods were loaded into a hanging rack of holders that were used to dip the rods continuously into media vials containing growth media. The rod heights were adjusted and each rod was secured in place using a rubber o-ring. In the early afternoon, 7mL of growth media (160g of a solution of 3% TSB with 3% sucrose was mixed with 240g pooled human saliva. This TSB/sucrose solution should be sterilized by autoclave before combining with the pooled human saliva.) into media vials. The media vials were arranged under the hanging rods on a rack in an incubation oven. The incubator has been previously modified such that a dipping motor can dip the rods into the media vials submerging 1.5 cm of the rod into the growth media at a frequency of 1 dip per minute without the rods touching the walls of the media vial. The rods were dipped overnight this way.

On Day 3, an enriched growth media was prepared (500g of a solution of 3% TSB and 10% sucrose was mixed with 33g pooled human saliva. This TSB/sucrose solution should be sterilized by autoclave before combining with the pooled human saliva.). This enriched growth media was pipetted into a new set of media vials (7mL per vial) and was swapped for the overnight growth media from Day 1. The rods were dipped throughout the day in this enriched growth media for 5 hours at 37°C in the incubation oven. At the end of the day, a new overnight growth media was prepared (40g of a solution of 3% TSB was mixed with 360g pooled human saliva and 0.5g sucrose), pipetted into a new set of media vials, and swapped for the enriched growth media. The rods were dipped overnight in the same fashion as on the first day.

On Day 4, a glycolysis media was prepared by combining 0.15g TSB, 25g sucrose, and 500 mL deionized water resulting in a solution of 0.03% TSB and 0.5% sucrose in water. This solution was mixed then sterilized in an autoclave. The pH was then adjusted to 6.5 using 0.1M HCl and pipetted into new media vials (7mL). Two extra vials were filled than were needed for the rack of rods as pH blanks. Two drops of chlorophenol red solution were added to each of the 4 tubes that contained the negative control (Crest Cavity Protection slurry). Three drops of bromocresol purple solution were added to 2 tubes that contained the positive control (1% Chlorhexidine solution). Set the rack aside until treatments are complete. Vials were prepared containing 12 mL of deionized water to rinse off the treatments. Vials were prepared containing the treatment slurries/solutions (7mL) of homogenized treatment and water. The rods were dipped into the treatment vials for 2 minutes, rinsed for 10 dips in a first set rinse vials, rinsed for 10 dips in a second set of rinse vials, rinsed for 10 dips in a third set of rinse vials, and returned to the incubator rack. The entire biofilm was treated and rinsed. Once all treatments were complete, the biofilms on the rods were fully submerged in the glycolysis media inside the incubation oven with no dipping for 2 hours. After two hours, the dipping apparatus was activated. The total incubation time was between 3 to 7 hours. Incubation is terminated when the pH value in the glycolysis media of the negative controls is between 4.8-5.6, more ideally 4.9-5.2, and when the pH value in the glycolysis media of the positive controls is above the negative control. If the indicator dye in the positive control turns yellow, i.e., the pH has dropped beneath 5.2, the incubation has gone on too long and the test will need to be repeated.

After incubation termination on Day 4, the rods were removed from the glycolysis media and allowed to dry in the oven. The glycolysis media was removed from the incubation oven, allowed to return to room temperature, and the pH was measured in each vial and the blank vials to determine the average pH change of the media following treatment. The change in pH is determined with respect to the blank vials. If the final pH of the blank is less than 6.6, the test needs to be

repeated. If the difference between the positive and negative control is not significant in a student's t-test, the test needs to be repeated. If the change in pH of the negative control with respect to the blank is less than 1, the test needs to be repeated.

After the pH values of all the vials were measured, the ΔpH per vial was determined by subtracting its pH from the average pH of the blanks. The glycolysis inhibition efficacy is determined from the following formula. The average ΔpH of a treatment was determined by averaging the results from the four replicate vials per treatment.

$$\text{Acid Inhibition (\%)} = 100 - \left(\frac{Avg\Delta pH_{sample}}{Avg\Delta pH_{neg\ ctrl}}\right) \times 100$$

Formula 1

If the efficacy of the positive control (1% Chlorhexidine solution) is not between about 65% to about 85% with respect to the negative control (Crest Cavity Protection, Procter & Gamble, Cincinnati, OH), the test was repeated.

TABLE 1A: Edible Oral Compositions

| Ex. 1 | Ex. 2 | Ex. 3 | Ex. 4 | Ex. 5 |
|---|---|---|---|---|
| **Bariatric Advantage Chew** | **Bariatric Advantage Chew plus Hops** | **Zolli Pops** | **Zolli Pops plus Hops** | **Vitafusion Calcium Chew** |
| Maltitol Syrup | Maltitol Syrup | Isomalt Syrup | Isomalt Syrup | Sugar |
| Palm Oil | Palm Oil | Erythritol | Erythritol | Glucose Syrup |
| Beet Juice Concentrate | Beet Juice Concentrate | Citric Acid | Citric Acid | Water |
| Natural Flavor | Natural Flavor | Natural Flavors | Natural Flavors | Gelatin |
| Citric Acid | Citric Acid | Natural Colors | Natural Colors | Canola Lecithin |
| Mono and Diglycerides | Mono and Diglycerides | Orange Oil | Orange Oil | Citric Acid |
| Soy Lecithin | Soy Lecithin | Stevia | Stevia | Natural Colors |
| Sea Salt | Sea Salt | | 0.5 % Hops Beta Acid Extract | Lactic Acid |
| Sucralose | Sucralose | | | Medium Chain Triglycerides |
| Calcium Citrate | Calcium Citrate | | | Natural Flavors |
| Cholecalciferol | Cholecalciferol | | | Pectin |
| | 0.5 % Hops Beta Acid Extract | | | Cholecalciferol |
| | | | | Tricalcium Phosphate |

TABLE 1B. Edible Oral Compositions

| Ex. 6 | Ex. 7 | Ex. 8 | Ex. 9 | Ex. 10 |
|---|---|---|---|---|
| **Vitafusion Calcium Chew plus Hops** | **Albanese Gummy Bear** | **Albanese Gummy Bear plus Hops** | **Basic Bites Chew** | **Basic Bites Chew plus Hops** |
| Sugar | Maltitol Syrup | Maltitol Syrup | Maltitol Syrup | Maltitol Syrup |
| Glucose Syrup | Water | Water | Calcium Carbonate | Calcium Carbonate |
| Water | Gelatin | Gelatin | Cacoa Powder | Cacoa Powder |

(continued)

| Ex. 6 | Ex. 7 | Ex. 8 | Ex. 9 | Ex. 10 |
|---|---|---|---|---|
| **Vitafusion Calcium Chew plus Hops** | **Albanese Gummy Bear** | **Albanese Gummy Bear plus Hops** | **Basic Bites Chew** | **Basic Bites Chew plus Hops** |
| Gelatin | Lactic Acid | Lactic Acid | Palm Oil | Palm Oil |
| Canola Lecithin | Natural and Artificial Flavors | Natural and Artificial Flavors | Natural Flavor | Natural Flavor |
| Citric Acid | Citric Acid | Citric Acid | Mono and Diglycerides | Mono and Diglycerides |
| Natural Colors | Pectin | Pectin | Soy Lecithin | Soy Lecithin |
| Lactic Acid | Vegetable Oil | Vegetable Oil | Arginine Bicarbonate | Arginine Bicarbonate |
| Medium Chain Triglycerides | Carnauba Leaf Wax | Carnauba Leaf Wax | Xylitol | Xylitol |
| Natural Flavors | Aspartame | Aspartame | | 0.5 % Hops Beta Acid Extract |
| Pectin | FD&C Yellow #5 | FD&C Yellow #5 | | |
| Cholecalciferol | FD&C Red #40 | FD&C Red #40 | | |
| Tricalcium Phosphate | FD&C Blue #1 | FD&C Blue #1 | | |
| 0.5 % Hops Beta Acid Extract | FD&C Yellow #6 | FD&C Yellow #6 | | |
| | | 0.5 % Hops Beta Acid Extract | | |

TABLE 1A and TABLE 1B list the ingredients in each edible composition that were then tested for their antimicrobial efficacy. Further, TABLE 1A and TABLE 1B display the specific amount of the hops beta acid extract added to the composition to enhance its antimicrobial and anticaries efficacy. The ingredients listed are typical of those in edible compositions and will be recognized by those skilled in the art. Examples 1, 3, 5, 7, and 9 as shown in TABLE 1A and TABLE 1B did not contain the Hops Beta Acid extract. Examples 2, 4, 6, 8, and 10 as shown in TABLE 1A and TABLE 1B contained the Hops Beta Acid extract. The Hops Beta Acids were supplied by Hopsteiner® as an extract from *Humulus lupulus.* The Hopsteiner® extract was approximately 45%, by weight of the extract, of hops beta acids and less than 1%, by weight of the extract, of hops alpha acids.

TABLE 2. Hops Beta Acids Extract Specification

| Ingredient | Amount (wt%) |
|---|---|
| Hops Beta Acids | $45 \pm 2$ |
| Hops Alpha Acids | $0.4 \pm 0.3$ |
| Hops oils | $1.5 \pm 0.5$ |
| Propylene Glycol | $20 \pm 15$ |
| Water | < 8% |
| pH | $11 \pm 0.5$ |

TABLE 2 describes the hops beta acid extract provided by Hopsteiner®. Since the hops beta acids are provided as an extract, there can be some variability in the amounts of certain ingredients. However, the extract comprises approximately 45 %, by weight of the extract, of the hops beta acids and approximately 0.4%, by weight of the extract, of hops alpha acids. This is dramatically different to previous hops extracts which typically have more hops alpha acids than hops beta acids. Other minor ingredients may be present in the Hops Beta Acid extract.

TABLE 3. Acid Production and Glycolysis Inhibition [%] after treatment of 3-day biofilm

| Treatment | Acid Production (ApH) | | Mean Acid Inhibition (%) | |
|---|---|---|---|---|
| | Mean | SE | Mean | SE |
| Bariatric Advantage Calcium Chew | 1.74 | 0.02 | -19.2 | 1.63 |
| Bariatric Advantage Calcium Chew plus Hops | 0.60 | 0.02 | 58.9 | 1.28 |
| Zolli Pops | 1.65 | 0.04 | -12.7 | 2.54 |
| Zolli Pops plus Hops | 0.46 | 0.01 | 68.8 | 0.91 |
| Vitafusion Calcium Chew | 1.68 | 0.05 | -15.1 | 3.21 |
| Vitafusion Calcium Chew plus Hops | 0.49 | 0.01 | 66.4 | 0.93 |
| Albanese Gummy Bear | 1.53 | 0.04 | -4.5 | 2.59 |
| Albanese Gummy Bear plus Hops | 0.38 | 0.03 | 74.1 | 1.82 |
| Basic Bites Chew | 1.76 | 0.04 | -20.5 | 2.68 |
| Basic Bites Chew plus Hops | *0.55* | 0.03 | 62.3 | 1.77 |
| Crest® Cavity Protection (NaF) | 1.46 | 0.03 | 0.0 | 2.39 |
| Crest® Gum Care ($SnF_2$ + $SnCl_2$) | 0.56 | 0.02 | 61.6 | 1.22 |

TABLE 3 displays the change in acid production in a 3-day biofilm using the iPGRM test described herein. After treatment with a commercial Crest® toothpaste containing sodium fluoride, the acid product mean is 1.50 with a mean inhibition of 0.0% because it is the reference condition for negligible antimicrobial efficacy. Treatment of the 3-day biofilm with a Crest® toothpaste containing stannous fluoride resulted in decreases in acid production and a net mean acid inhibition of 66.9% (Crest® Gum Care). This was an expected result as the stannous ion is known to act as an antibacterial, which can lower the number of bacteria producing acid in the biofilm. Crest® Gum Care performed has two sources of stannous ions (i.e. $SnF_2$ and $SnCl_2$).

TABLE 3 also shows that the commercial edible compositions without hops all performed worse than the reference Crest® Cavity Protection toothpaste meaning that the biofilm produced more acid than the negligible antimicrobial control. It was unexpected that by adding hops beta acid extract to the commercial edible compositions that it increased that antimicrobial efficacy of every composition. Interestingly, the Hops Beta Acid extract was able to suppress plaque acid formation of the Vitafusion Calcium Chew even though the commercial edible composition contained the fermentable carbohydrates sugar and glucose syrup. These compounds are nutrient sources for the microorganisms, so it was further unexpected that the Hops Beta Acid extract was effective as an antimicrobial in the composition when there was also a food source included in the edible composition.

The hops beta acid extract and the hops total extract were added to Basic Bites Chew, which included arginine bicarbonate. The addition of the hops beta acid extract to Basic Bites Chew improved the acid inhibition from -20.5% to 62.3%. In the other formulas where hops was added, the average effect of the hops was an additional 80% acid inhibition. However, in the Basic Bites Chew formula with hops, the additional benefit was nearly 83%, a 3% increase in acid inhibition. The Basic Bites Chew formula has the basic amino acid, arginine. It is well known that arginolytic bacteria that can consume arginine would nevertheless prefer to consume sugar and make acid in the presence of a sugar source and at low pH, namely the conditions of the iPGRM used here. Therefore, in order for bacteria to effectively utilize arginine to produce ammonia and neutralize acid-forming biofilms, it is required that the biofilm pH be maintained at quite a high level already. This tends to impede arginine's effectiveness in biofilms where sugar is present and the pH is low. As demonstrated in TABLE 3, hops activates that arginolytic bacterial pathway by preventing the pH from falling in the presence of sugar. This, unexpectedly, produced a synergistic effect and resulted in a more effectively stabilized biofilm pH than either ingredient could achieve on its own.

Thus, hops beta acids are an extremely effective antibacterial agent, which can improve existing edible compositions regardless of chassis. Hops beta acids can improve antibacterial activity in edible compositions comprising fermentable carbohydrates, non-fermentable sugar alcohols, natural sweeteners (e.g., stevia) and/or artificial sweeteners (e.g., sucralose). Hops beta acids can improve antibacterial activity in edible compositions comprising calcium, such as calcium carbonate or tricalcium phosphate. Hops beta acids can improve antibacterial activity in edible compositions comprising an amino acid, such as arginine. Hops beta acids can improve antibacterial activity in edible compositions with a pH of 7, greater than 7, or less than 7.

Thus, described herein are edible compositions with a mean acid inhibition of at least 50%, at least 55%, at least 60%, at least 70%, or at least 80%, upon the addition of hops beta acid, either through a hops beta acid extract, a direct

addition of one or more hops beta acids, or any other suitable source of hops beta acid with respect to their hops-free composition.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An edible oral composition comprising:

   (a) hops, preferably wherein the edible oral composition comprises from 0.01% to 10%, by weight of the oral composition, of the hops, wherein the hops comprises hops beta acid and wherein the hops beta acid is free of hydrogentated hops beta acid; and
   (b) calcium, wherein the calcium comprises calcium salt, calcium abrasive, or combinations thereof, wherein the calcium abrasive comprises calcium carbonate, calcium pyrophosphate, or combinations thereof, and wherein the calcium salt comprises calcium chloride, calcium citrate, or combinations thereof.

2. The edible oral composition of claim 1, wherein the edible oral composition further comprises tin, and/or sugar alcohol.

3. The edible oral composition of any one of claims 1 or 2, wherein the hops beta acid comprises lupulone, adlupulone, colupulone, or combinations thereof, preferably wherein the hops comprises at least 35%, by weight of the hops, of hops beta acid, more preferably wherein the hops comprises less than 1%, by weight of the hops, of hops alpha acid.

4. The edible oral composition of any one of claims 1 to 3, wherein the edible oral composition comprises vitamin, preferably wherein the vitamin comprises vitamin A, vitamin B, vitamin C, vitamin D, vitamin E, or combinations thereof, more preferably wherein the Vitamin A comprises retinoid compound.

5. The edible oral composition of anyone of claims 1 to 4, wherein the edible oral composition comprises amino acid, preferably wherein the amino acid comprises arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutonic acid, diaminoproprionic acid, salts thereof, or combinations thereof.

6. The edible oral composition of claim 5, wherein the amino acid comprises peptide, polypeptide, or combinations thereof, preferably wherein the peptide comprises from two amino acids to ten amino acids, more preferably wherein the peptide comprises a dipeptide, a tripeptide, a tetrapeptide, a pentapeptide, or combinations thereof, even more preferably wherein the pentapeptide comprises Pal-KTTKS.

7. The edible oral composition of any one of claims 2 to 6, wherein the tin comprises stannous fluoride, stannous chloride, or combinations thereof.

8. The edible oral composition of any one of claims 1 to 7, wherein the edible oral composition comprises fluoride, preferably wherein the fluoride comprises sodium fluoride, sodium monofluorophosphate, stannous fluoride, amine fluoride, or combinations thereof, more preferably wherein the edible oral composition provides fluoride ions at a concentration of up to 1 $\pm$ 10% ppm, up to 0.7 $\pm$ 10% ppm, or less than 0.7 $\pm$ 10% ppm.

9. The edible oral composition of any one of claims 2 to 8, wherein the sugar alcohol comprises xylitol, erythritol, sorbitol, malitol, isomalt, or combinations thereof.

10. The edible oral composition of any one of claims 1 to 9, wherein the composition is free of sucrose.

11. The edible oral composition of any one of claims 1 to 10, wherein the edible oral composition is a gummy composition, a chewable composition, a pan-chew composition, a tablet composition, a dissolving tablet composition, a dissolving film, a leave-on composition, a lollipop composition, a lozenge composition, a nonwoven fiber composition, a soluble foam composition, a liquid composition, a paste composition, chewing gum composition, or combinations thereof.

**Patentansprüche**

1. Verzehrbare orale Zusammensetzung, umfassend:

   (a) Hopfen, vorzugsweise wobei die verzehrbare orale Zusammensetzung von zu 0,01 Gew.-% bis 10 Gew.-%, der oralen Zusammensetzung, Hopfen umfasst, wobei der Hopfen Hopfenbetasäure umfasst und wobei die Hopfenbetasäure frei von hydrierter Hopfenbetasäure ist; und
   (b) Calcium, wobei das Calcium Calciumsalz, Calciumschleifmittel oder Kombinationen davon umfasst, wobei das Calciumschleifmittel Calciumcarbonat, Calciumpyrophosphat oder Kombinationen davon umfasst und wobei das Calciumsalz Calciumchlorid, Calciumcitrat oder Kombinationen davon umfasst.

2. Verzehrbare orale Zusammensetzung nach Anspruch 1, wobei die verzehrbare orale Zusammensetzung ferner Zinn und/oder Zuckeralkohol umfasst.

3. Verzehrbare orale Zusammensetzung nach einem der Ansprüche 1 oder 2, wobei die Hopfenbetasäure Lupulon, Adlupulon, Colupulon oder Kombinationen davon umfasst, wobei der Hopfen vorzugsweise wenigstens zu 35 Gew.-% des Hopfens Hopfenbetasäure umfasst, mehr bevorzugt wobei der Hopfen weniger als zu 1 Gew.-% des Hopfens Hopfenalphasäure umfasst.

4. Verzehrbare orale Zusammensetzung nach einem der Ansprüche 1 bis 3, wobei die verzehrbare orale Zusammensetzung Vitamin umfasst, vorzugsweise wobei das Vitamin Vitamin A, Vitamin B, Vitamin C, Vitamin D, Vitamin E oder Kombinationen davon umfasst, mehr bevorzugt wobei das Vitamin A eine Retinoidverbindung umfasst.

5. Verzehrbare orale Zusammensetzung nach einem der Ansprüche 1 bis 4, wobei die verzehrbare orale Zusammensetzung eine Aminosäure umfasst, vorzugsweise wobei die Aminosäure Arginin, Histidin, Lysin, Asparaginsäure, Glutaminsäure, Serin, Threonin, Asparagin, Glutamin, Cystein, Selenocystein, Glycin, Prolin, Alanin, Valin, Isoleucin, Leucin, Methionin, Phenylalanin, Tyrosin, Tryptophan, Citrullin, Ornithin, Kreatin, Diaminobutansäure, Diaminopropionsäure, Salze davon oder Kombinationen davon umfasst.

6. Verzehrbare orale Zusammensetzung nach Anspruch 5, wobei die Aminosäure Peptid, Polypeptid oder Kombinationen davon umfasst, vorzugsweise wobei das Peptid von zwei Aminosäuren bis zehn Aminosäuren umfasst, mehr bevorzugt wobei das Peptid ein Dipeptid, ein Tripeptid, ein Tetrapeptid, ein Pentapeptid oder Kombinationen davon umfasst, noch mehr bevorzugt wobei das Peptid Pal-KTTKS umfasst.

7. Verzehrbare orale Zusammensetzung nach Anspruch 2, wobei das Zinn Zinnfluorid, Zinn(II)-chlorid oder Kombinationen davon umfasst.

8. Verzehrbare orale Zusammensetzung nach einem der Ansprüche 1 bis 7, wobei die verzehrbare orale Zusammensetzung Fluorid umfasst, vorzugsweise wobei das Fluorid Natriumfluorid, Natriummonofluorphosphat, Zinnfluorid, Aminfluorid oder Kombinationen davon umfasst, mehr bevorzugt wobei die verzehrbare orale Zusammensetzung Fluoridionen in einer Konzentration von bis zu 1 $\pm$ 10 % ppm, bis zu 0,7 $\pm$ 10 % ppm oder weniger als 0,7 $\pm$ 10 % ppm bereitstellt.

9. Verzehrbare orale Zusammensetzung nach einem der Ansprüche 2 bis 8, wobei der Zuckeralkohol Xylitol, Erythritol, Sorbitol, Malitol, Isomalt oder Kombinationen davon umfasst.

10. Verzehrbare orale Zusammensetzung nach einem der Ansprüche 1 bis 9, wobei die Zusammensetzung frei von Saccharose ist.

11. Verzehrbare orale Zusammensetzung nach einem der Ansprüche 1 bis 10, wobei die verzehrbare orale Zusammensetzung eine Gummizusammensetzung, eine kaubare Zusammensetzung, eine Pan-Kauzusammensetzung, eine Tablettenzusammensetzung, eine Schmelztablettenzusammensetzung, eine Schmelzfolie, eine zum Aufbehalten bestimmte Zusammensetzung, eine Lutscherzusammensetzung, eine Lutschtablettenzusammensetzung, eine Vliesfaserzusammensetzung, eine lösliche Schaumzusammensetzung, eine flüssige Zusammensetzung, eine Pastenzusammensetzung, eine Kaugummizusammensetzung oder Kombinationen davon ist.

**Revendications**

1. Composition orale comestible comprenant :

   (a) du houblon, de préférence dans laquelle la composition orale comestible comprend de 0,01 % à 10 %, en poids de la composition orale, du houblon, dans laquelle le houblon comprend un acide bêta de houblon et dans laquelle l'acide bêta de houblon est exempt d'acide bêta de houblon hydrogéné ; et
   (b) du calcium, dans laquelle le calcium comprend un sel de calcium, un abrasif de calcium, ou des combinaisons de ceux-ci, dans laquelle l'abrasif de calcium comprend du carbonate de calcium, du pyrophosphate de calcium, ou des combinaisons de ceux-ci, et dans laquelle le sel de calcium comprend du chlorure de calcium, du citrate de calcium, ou des combinaisons de ceux-ci.

2. Composition orale comestible selon la revendication 1, dans laquelle la composition orale comestible comprend en outre de l'étain, et/ou de l'alcool de sucre.

3. Composition orale comestible selon l'une quelconque des revendications 1 ou 2, dans laquelle l'acide bêta de houblon comprend lupulone, adlupulone, colupulone, ou des combinaisons de celles-ci, de préférence dans laquelle le houblon comprend au moins 35 %, en poids du houblon, d'acide bêta de houblon, plus préférablement dans laquelle le houblon comprend moins de 1 %, en poids du houblon, d'acide alpha de houblon.

4. Composition orale comestible selon l'une quelconque des revendications 1 à 3, dans laquelle la composition orale comestible comprend des vitamines, de préférence dans laquelle la vitamine comprend vitamine A, vitamine B, vitamine C, vitamine D, vitamine E, ou des combinaisons de celles-ci, plus préférablement dans laquelle la vitamine A comprend un composé rétinoïde.

5. Composition orale comestible selon l'une quelconque des revendications 1 à 4, dans laquelle la composition orale comestible comprend un acide aminé, de préférence dans laquelle l'acide aminé comprend arginine, histidine, lysine, acide aspartique, acide glutamique, sérine, thréonine, asparagine, glutamine, cystéine, sélénocystéine, glycine, proline, alanine, valine, isoleucine, leucine, méthionine, phénylalanine, tyrosine, tryptophane, citrulline, ornithine, créatine, acide diaminobutonique, acide diaminopropionique, sels de ceux-ci, ou combinaisons de ceux-ci.

6. Composition orale comestible selon la revendication 5, dans laquelle l'acide aminé comprend un peptide, un polypeptide, ou des combinaisons de ceux-ci, de préférence dans laquelle le peptide comprend de deux acides aminés à dix acides aminés, plus préférablement dans laquelle le peptide comprend un dipeptide, un tripeptide, un tétrapeptide, un pentapeptide, ou des combinaisons de ceux-ci, même plus préférablement dans laquelle le pentapeptide comprend Pal-KTTKS.

7. Composition orale comestible selon l'une quelconque des revendications 2 à 6, dans laquelle l'étain comprend du fluorure stanneux, du chlorure stanneux, ou des combinaisons de ceux-ci.

8. Composition orale comestible selon l'une quelconque des revendications 1 à 7, dans laquelle la composition orale comestible comprend du fluorure, de préférence dans laquelle le fluorure comprend fluorure de sodium, monofluorophosphate de sodium, fluorure stanneux, fluorure d'amine, ou des combinaisons de ceux-ci, plus préférablement dans laquelle la composition orale comestible fournit des ions fluorure à une concentration allant jusqu'à 1 $\pm$ 10 % ppm, jusqu'à 0,7 $\pm$ 10 % ppm, ou moins de 0,7 $\pm$ 10 % ppm.

9. Composition orale comestible selon l'une quelconque des revendications 2 à 8, dans laquelle l'alcool de sucre comprend xylitol, érythritol, sorbitol, malitol, isomalt, ou des combinaisons de ceux-ci.

10. Composition orale comestible selon l'une quelconque des revendications 1 à 9, dans laquelle la composition est exempte de saccharose.

11. Composition orale comestible selon l'une quelconque des revendications 1 à 10, dans laquelle la composition orale comestible est une composition de gomme, une composition à mâcher, une composition à mâcher en plaque, une composition de comprimé, une composition de comprimé soluble, un film soluble, une composition à laisser en place, une composition de sucette, une composition de pastille, une composition de fibres non tissées, une composition de mousse soluble, une composition liquide, une composition de pâte, une composition de chewing-gum, ou des combinaisons de celles-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015171837 A1
- US 7910140 B
- US 5370863 A
- US 4154813 A
- US 5762911 A
- US 5180577 A
- US 20170360865
- US 10849729 B
- US 2018013319
- US 10780032 B
- US 10932996 B
- US 5340580 A, Barbera
- US 4548806 A
- US 4459280 A, Colliopoulos

### Non-patent literature cited in the description

- IUPAC Compendium of Chemical Terminology. 1997
- *Chemical and Engineering News*, 1985, vol. 63 (5), 27